# EUROPEAN PATENT APPLICATION

(11) **EP 4 375 278 A1**
(43) Date of publication of application: **29.05.2024**
(21) Application number: 22853458.2
(22) Date of filing: 03.08.2022
(51) Int. Cl.: C07D 401/04, C07H 17/02, A61K 31/454, A61K 31/706, A61K 9/14, A61K 47/26, A61K 9/00, A61P 35/00, A61P 29/00, A61P 17/06

(54) **IMMUNOREGULATORY AMIDE DERIVATIVE WITH IMPROVED MATERIAL PROPERTIES**

(30) Priority: 04.08.2021 KR 20210102394
(71) Applicant: Aevis Bio, Inc., Daejeon 34141 (KR)
(72) Inventor: KIM, Dong Seok, Daejeon 34033 (KR); HWANG, Inho, Cheonan-si, Chungcheongnam-do 31145 (KR); KIM, Sun, Daejeon 34118 (KR)
(74) Representative: Turner, Craig Robert
(86) International application number: PCT/KR2022/011480
(87) International publication number: WO 2023/014082

(57) **Abstract**

The present invention relates to a novel thalidomide-based compound derivative having significantly improved water solubility due to the introduction of a sugar or sugar derivative into lenalidomide or pomalidomide, which are thalidomide-based compounds, wherein the lenalidomide or pomalidomide derivative according to the present invention has significantly increased water solubility compared to conventional lenalidomide or pomalidomide and can thus be formulated into an injection or eye drop formulation. Therefore, the lenalidomide or pomalidomide derivative can be administered in the form of a high concentration therapeutic agent to a local area, and can be easily administered in combination with a different therapeutic agent developed as an injection or eye drop formulation.

## Description

### [Technical Field]

The present invent ion relates to an immunoregulatory amide derivative with improved material properties, and more particularly, to a lenalidomide or pomalidomide derivative having significantly improved water solubility due to the introduction of a sugar or sugar derivative into a thalidomide-based compound such as lenalidomide or pomalidomide.

### [Background Art]

Thalidomide was a drug sold from the late 1950s to the 1960s to prevent morning sickness in pregnant women, but as teratogenic side effects were reported, its use and handling were prohibited for pregnant women as well as people of childbearing age or those who may become pregnant. However, as the therapeutic effect of thalidomide on multiple myeloma and the mechanism of its side effects have recently revealed, research on the development of a therapeutic agent for multiple myeloma using a thalidomide-based compound is actively progressing.

Multiple myeloma is a blood cancer that occurs when plasma cells abnormally differentiate and proliferate, and such abnormal plasma cells are called myeloma cells. Myeloma cells form tumors, dissolve bones, cause pain, make bone more brittle, and invade the bone marrow to reduce white blood cells, red blood cells, and platelet levels, increasing the risk of anemia, infection, and bleeding. In addition, myeloma cells produce M proteins, which is an abnormal immune protein, which thickens the concentration of blood, causing blood hyper viscosity syndrome or damaging the kidneys. It mainly occurs in blacks, men, people over the age of 65, and in Korea, recently, the frequency of occurrence has been gradually increasing. The main therapeutic agents for multiple myeloma include bortezomib, thalidomide, lenalidomide, and pomalidomide. The injectable drug, bortezomib, is steadily maintaining its market share and growing, and as an oral agent, lenalidomide is replacing the position of existing thalidomide, and is recording high growth in the market.

Lenalidomide is the next-generation drug of thalidomide and shows better therapeutic effects than thalidomide through more powerful killing of cancer cells and immunoregulation. In cases of recurrence or refractoriness to existing treatment, in treatment with a combination of lenalidomide and dexamethasone, it is known that a disease-free survival period is 13.4 months, and an overall survival period is 38 months, which is known to be quite effective. As for side effects, a problem such as peripheral neuropathy that occurred with existing thalidomide has almost disappeared, and the side effect of bone marrow suppression has been slightly more severe, but it is known that there is no major problem when administering leukocyte stimulating factors.

Pomalidomide, approved by the US FDA in 2013 as a therapeutic agent for recurrent and refractory multiple myeloma, is used when the disease progresses within 60 days after the final treatment in people who have previously been treated with at least two therapeutic agents, including lenalidomide and bortezomib. Pomalidomide directly inhibits angiogenesis and myeloma cell growth, and this dual effect is central to the activity of pomalidomide in myeloma rather than to other pathway such as TNF-α inhibition. Through not only the enhancement of IFN-γ, IL-2 and IL-10 expression but also the suppression of IL-6 expression, pomalidomide exhibits anti-angiogenic and anti-myeloma activities.

Both lenalidomide and pomalidomide were developed as oral capsule preparations by Celgene, and commercialized and used in doses of 1 to 25 mg. The brand name of lenalidomide released in Korea is Revlimid^{®} Capsule, and the brand name of pomalidomide released in Korea is Pomalyst^{®} Capsule, and these are all filled in capsule No. 0, which has a long axis of about 2.17 cm, a significantly long length, and a large volume.

Therefore, patients, particularly, elderly patients, may experience a little inconvenience in dosing, and in addition, even when being taken with water, the capsule may stick to the throat or esophagus and become stuck during swallowing. Here, there may be a case where the capsule does not fall even after drinking a large amount of water, or it is painful when the drug accidently bursts, and attempts have been made to improve the physical properties of oral preparations, considering the various problems that inflammation may generate in some cases (Korean Unexamined Patent Application Publication No. 10- 2020-0013258).

However, the above related art was intended to improve the physical properties of lenalidomide or pomalidomide as an oral preparation, wherein lenalidomide or pomalidomide was not dissolved in water and thus it was difficult to develop them as injectable drugs. Therefore, when these therapeutic agents were administered in combination with another therapeutic agent (particularly, a therapeutic agent administered intravenously), since these are administered orally unlike other therapeutic agents administered intravenously, the administration method is complex in that the therapeutic agents are administered in different ways, and in oral administration, an injection is difficult to be administered locally at a high concentration.

Therefore, the present inventors have intensively tried to develop a novel compound having improved physical properties such that it can be used in various formulations such as injections, eye drops, sprays, patches, etc. while maintaining the same or higher effect compared with lenalidomide or pomalidomide, as a result, it was confirmed that, when a sugar or sugar derivative is introduced to an amino group (NH₂) present in the phthalimide moiety of lenalidomide or pomalidomide, the pharmaceutical efficacy of lenalidomide or pomalidomide is maintained, water solubility can significantly increase, and the teratogenic side effects caused by lenalidomide or pomalidomide can be reduced. Thus, the present invention was completed.

### [Disclosure]

### [Technical Problem]

The present invention is directed to providing an immunoregulatory amide derivative having improved material properties such as improved water solubility so that it can be used in an injection or eye drops.

### [Technical Solution]

One aspect of the present invention provides a lenalidomide or pomalidomide derivative in which a sugar or sugar derivative is bound to an amino group (NH₂) on the phthalimide moiety of lenalidomide or pomalidomide.

In the present invention, the sugar or sugar derivative may be a triose, a tetrose, a pentose, a hexose, or a derivative thereof.

In the present invention, the sugar or sugar derivative may be glucose, ribose, glucuronic acid, or glycerin aldehyde.

In the present invention, the derivative may be selected from the group consisting of Formulas III to XIV below: 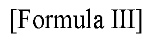 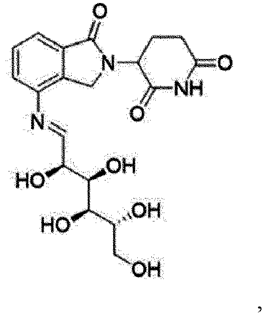 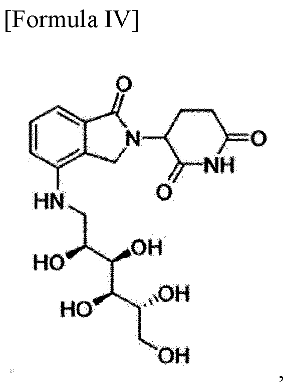 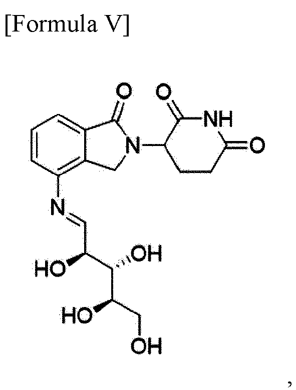 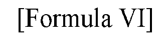 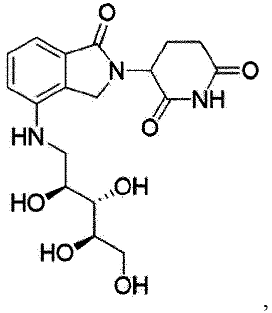 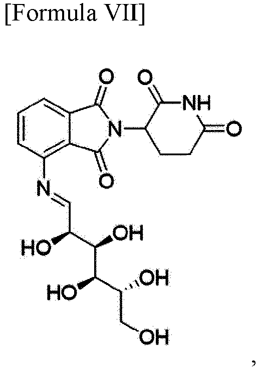 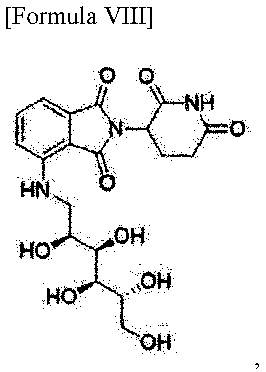 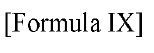 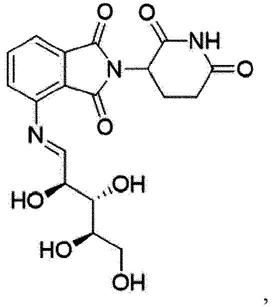 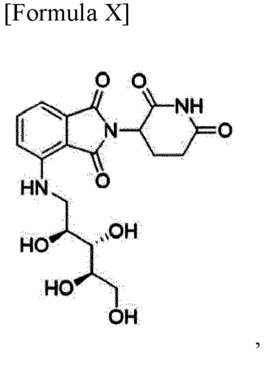 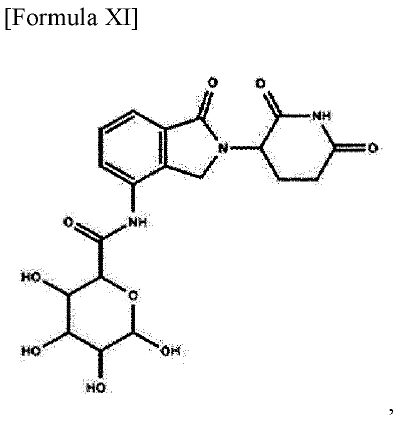 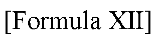 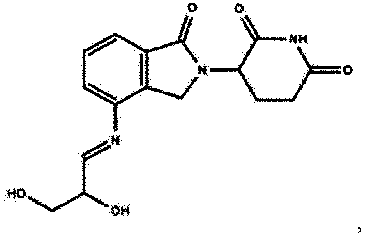 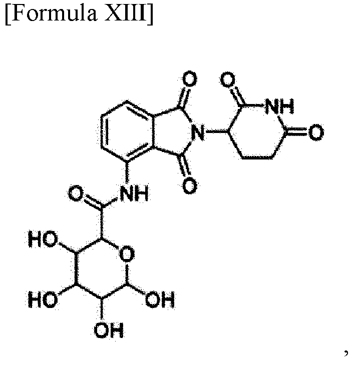 and

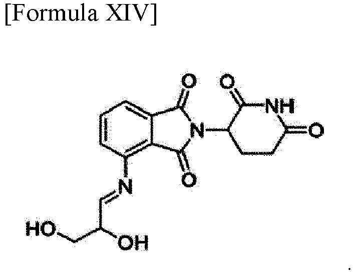

In the present invention, the derivative may have water solubility improved by more than 50% compared to lenalidomide or pomalidomide.

Another aspect of the present invention provides a pharmaceutical composition for preventing or treating cancer, which includes the derivative as an active ingredient.

Still another aspect of the present invention provides a method of preventing or treating cancer, which includes administering the derivative to a subject in need thereof.

Yet another aspect of the present invention provides the use of the derivative for preventing or treating cancer.

Yet another aspect of the present invention provides the use of the derivative for preparing a drug for preventing or treating cancer.

In the present invention, the cancer may be blood cancer or solid cancer.

In the present invention, the blood cancer may be selected from the group consisting of acute leukemia, chronic leukemia, multiple myeloma, Hodgkin's lymphoma, and non-Hodgkin's lymphoma.

Yet another aspect of the present invention provides a pharmaceutical composition for preventing or treating a myelodysplastic syndrome, which includes the derivative as an active ingredient.

Yet another aspect of the present invention provides a method of preventing or treating a myelodysplastic syndrome, which includes administering the derivative to a subject in need thereof.

Yet another aspect of the present invention provides the use of the derivative for preventing or treating a myelodysplastic syndrome.

Yet another aspect of the present invention provides the use of the derivative for preparing a drug for preventing or treating a myelodysplastic syndrome.

Yet another aspect of the present invention provides a pharmaceutical composition for preventing or treating an inflammatory disease, which includes the derivative as an active ingredient.

Yet another aspect of the present invention provides a method of preventing or treating an inflammatory disease, which includes administering the derivative to a subject in need thereof.

Yet another aspect of the present invention provides the use of the derivative for preventing or treating an inflammatory disease.

Yet another aspect of the present invention provides the use of the derivative for preparing a drug for preventing or treating an inflammatory disease.

In the present invention, the inflammatory disease may be selected from the group consisting of psoriasis, rheumatoid arthritis, and Crohn's disease.

Yet another aspect of the present invention provides a pharmaceutical composition for preventing or treating a brain disease, which includes the derivative as an active ingredient.

Yet another aspect of the present invention provides a method of preventing or treating a brain disease, which includes administering the derivative to a subject in need thereof.

Yet another aspect of the present invention provides the use of the derivative for preventing or treating a brain disease.

Yet another aspect of the present invention provides the use of the derivative for preparing a drug for preventing or treating a brain disease.

In the present invention, the brain disease may be selected from the group consisting of traumatic brain injury, Alzheimer's disease, Parkinson's disease, Huntington's disease, Lou Gehrig's disease, multiple system atrophy, Alzheimer's dementia, vascular dementia, frontotemporal dementia, Lewy dementia, ischemic stroke, hemorrhagic stroke, and multiple sclerosis.

Yet another aspect of the present invention provides a pharmaceutical composition for preventing or treating an angiogenic disease, which includes the derivative as an active ingredient.

Yet another aspect of the present invention provides a method of preventing or treating an angiogenic disease, which includes administering the derivative to a subject in need thereof.

Yet another aspect of the present invention provides the use of the derivative for preventing or treating an angiogenic disease.

Yet another aspect of the present invention provides the use of the derivative for preparing a drug for preventing or treating an angiogenic disease.

In the present invention, the angiogenic disease may be selected from the group consisting of corneal transplant angiogenesis, angiogenic glaucoma, diabetic retinopathy, macular degeneration, diabetic macular edema, a corneal disease caused by new blood vessels, spot degeneration, pterygium, retinal degeneration, retrolental fibroplasia, granular conjunctivitis, hemangioma, angiofibroma, vascular malformation, arteriosclerosis, vascular adhesion, and edematous sclerosis.

Yet another aspect of the present invention provides an injection preparation containing the derivative.

Yet another aspect of the present invention provides an eye drop preparation containing the derivative.

### [Advantageous Effects]

The lenalidomide or pomalidomide derivative according to the present invention has significantly increased water solubility compared to conventional lenalidomide or pomalidomide and can thus be formulated into an injection or eye drop preparation. Therefore, the lenalidomide or pomalidomide derivative can be administered in the form of a high concentration therapeutic agent to a local area and can be easily administered in combination with a different therapeutic agent developed as an injection or eye drop formulation. Additionally, it has the effect of reducing the teratogenic side effects compared to existing lenalidomide or pomalidomide.

### [Description of Drawings]

FIG. 1 shows the structural formulas of (A) thalidomide, (B) lenalidomide, and (C) pomalidomide.
FIG. 2a shows the chemical formula of ribose-lenalidomide.
FIG. 2b shows the solubility of ribose-lenalidomide in D.W.
FIG. 2c shows the CRBN binding affinity of ribose-lenalidomide.
FIG. 2d shows the results that confirm the tumor cell killing effect of ribose-lenalidomide in an MM.1S cell line, which is a human multiple myeloma cell line.
FIG. 2e shows the results that confirm the degradation ability of ribose-lenalidomide for Aiolos, Ikaros, CK1α in an MM.1S cell line, which is a human multiple myeloma cell line.
FIG. 2f shows the results that confirm the SALL4 degradation ability of ribose-lenalidomide in an HTB-105 cell line, which is a human embryonic carcinoma Tera-1 cell line.
FIG. 2g shows the results that confirm the anti-inflammatory effect of ribose-lenalidomide at the mRNA level using a PBMC cell line, which is a human peripheral blood mononuclear cell line.
FIG. 2h shows the results that confirm the anti-inflammatory effect of ribose-lenalidomide at the protein level using a PBMC cell line, which is a human peripheral blood mononuclear cell line.
FIG. 2i shows the results that confirm the macular degradation treatment effect of ribose-lenalidomide according to direct intravitreal administration and administration of eye drops using a macular degradation mouse model.
FIG. 3a shows the chemical formula of glucose-lenalidomide.
FIG. 3b shows the solubility of glucose-lenalidomide in D.W.
FIG. 3c shows the CRBN binding affinity of glucose-lenalidomide.
FIG. 3d shows the results that confirm the tumor cell killing effect of glucose-lenalidomide in an MM.1S cell line, which is a human multiple myeloma cell line.
FIG. 3e shows the results that confirm the SALL4 degradation ability of glucose-lenalidomide in an HTB-105 cell line, which is a human embryonic carcinoma Tera-1 cell line.
FIG. 3f shows the results that confirm the macular degradation treatment effect of ribose-lenalidomide according to direct intravitreal administration using a macular degradation mouse model (*p<0.05 vs CNV(+Vehicle)).
FIG. 4a shows the chemical formula of ribose-pomalidomide.
FIG. 4b shows the solubility of ribose-pomalidomide in D.W.
FIG. 4c shows the CRBN binding affinity of ribose-pomalidomide.
FIG. 4d shows the results that confirm the tumor cell killing effect of ribose-pomalidomide in an MM.1S cell line, which is a human multiple myeloma cell line.
FIG. 4e shows the results that confirm the SALL4 degradation ability of ribose-pomalidomide in an HTB-105 cell line, which is a human embryonic carcinoma Tera-1 cell line.
FIG. 5a shows the chemical formula of glucose-pomalidomide.
FIG. 5b shows the solubility of glucose-pomalidomide in D.W.
FIG. 5c shows the CRBN binding affinity of glucose-pomalidomide.
FIG. 5d shows the results that confirm the tumor cell killing effect of glucose-pomalidomide in an MM.1S cell line, which is a human multiple myeloma cell line.

### [Modes of the Invention]

Unless defined otherwise, all technical and scientific terms used in the specification have the same meanings as commonly understood by one of ordinary skill in the art to which the present invention belongs. Generally, the nomenclature used herein and the experimental methods to be described below are well known in the art and commonly used.

In the present invention, when an appropriate sugar or sugar derivative is introduced to an amino group present on the phthalimide moiety of a thalidomide-based compound such as lenalidomide or pomalidomide, it was confirmed that the water solubility of lenalidomide or pomalidomide is significantly improved, and the drug efficacy of the thalidomide-based compound is maintained.

Accordingly, in one aspect, the present invention relates to lenalidomide or pomalidomide derivative in which a sugar or sugar derivative is bound to an amino group (NH₂) on the phthalimide moiety of lenalidomide or pomalidomide.

Lenalidomide is known as (RS)-3-(4-amino-1-oxo-1,3-dihydro-2H-isoindol-2-yl)piperidine-2,6-dione, and has Formula (I) below:

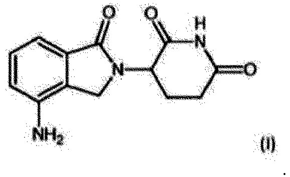

Pomalidomide is known as (RS)-4-amino-2-(2,6-dioxopiperidin-3-yl)isoindol-1,3-dione, and has Formula (II) below:

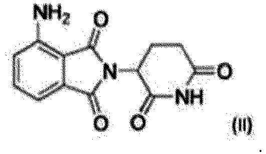

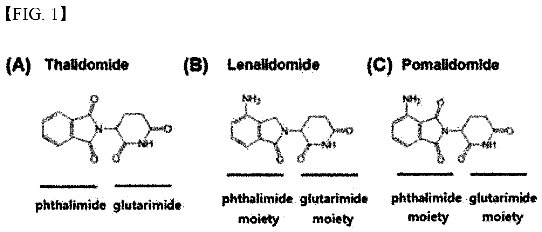

Pomalidomide is a compound in which an amino group is added to the phthalimide of thalidomide, and lenalidomide has a pomalidomide structure in which one carbonyl group is removed from the phthalimide moiety (see FIG. 1). These two compounds are immunomodulatory drugs (IMiD) and are reported to have a stronger immunomodulatory activity than thalidomide.

Lenalidomide and pomalidomide each have a phthalimide moiety and a glutarimide moiety (see FIG. 1), and in the present invention, the sugar or sugar derivative is bound to an amino group on the phthalimide moiety.

In the present invention, the sugar or sugar derivative may be a triose, a tetrose, a pentose, a hexose, or a derivative thereof, but the present invention is not limited thereto.

In some embodiments of the present invention, the sugar may be glucose, ribose, glucuronic acid, or glycerin aldehyde, but the present invention is not limited thereto.

In the present invention, the derivative may be selected from the group consisting of Formulas III to XIV below, but the present invention is not limited thereto: 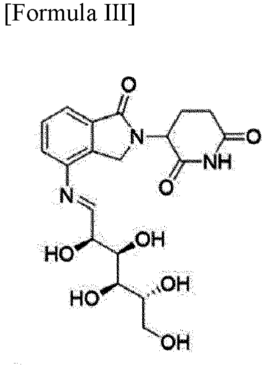 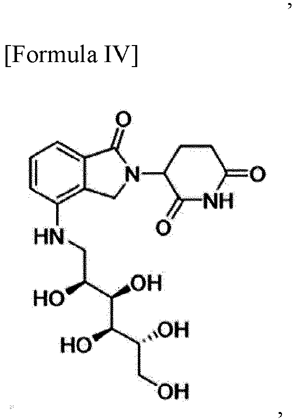 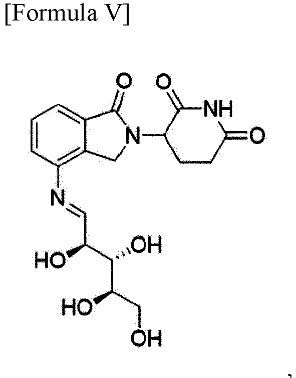 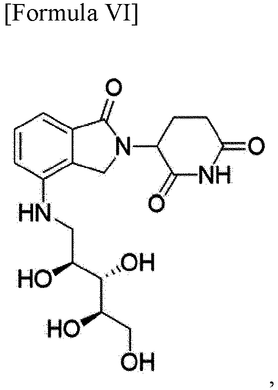 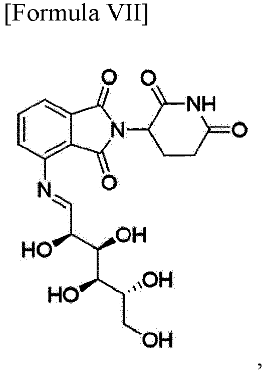 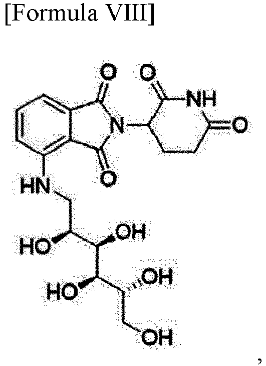 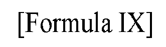 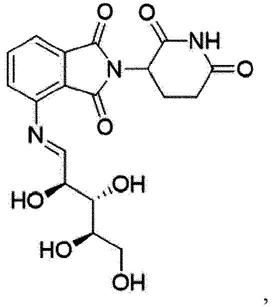 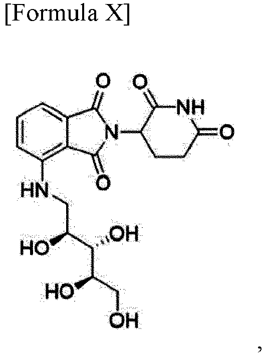 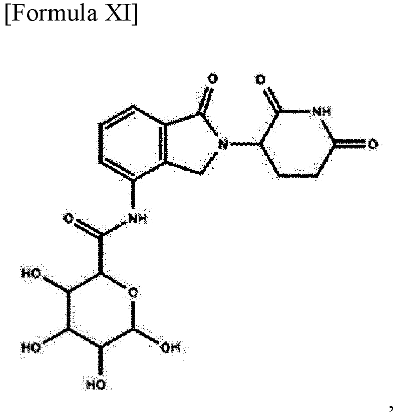 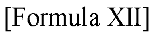 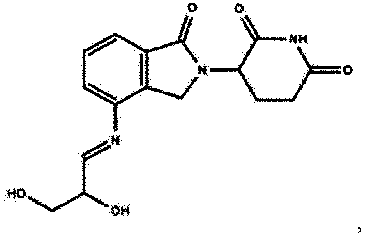 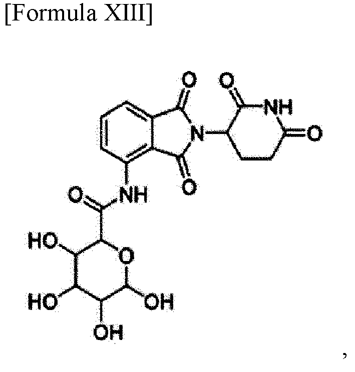 and

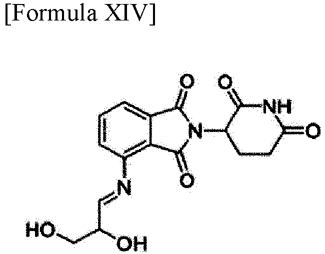

In the present invention, the derivative represented by Formula III and the derivative represented by Formula IV are two types in which glucose is bound to lenalidomide.

In the present invention, the derivative represented by Formula V or the derivative represented by Formula VI is one in which ribose is bound to lenalidomide.

In the present invention, the derivative represented by Formula VII and the derivative represented by Formula VIII are two types in which glucose is bound to pomalidomide.

In the present invention, the derivative represented by Formula IX, and the derivative represented by Formula X are two types in which ribose is bound to pomalidomide.

Meanwhile, in the present invention, the derivative represented by Formula XI is one in which glucuronic acid is bound to lenalidomide, and the derivative represented by Formula XII is one in which glycerin aldehyde is bound to lenalidomide, the derivative represented by Formula XIII is one in which glucuronic acid is bound to pomalidomide, and the derivative represented by Formula XIV is one in which glycerin aldehyde is bound to pomalidomide.

In the present invention, the derivative may have improved water solubility compared to lenalidomide or pomalidomide, and the water solubility may be improved by about 10% or more, preferably about 20% or more, more preferably about 30% or more, and most preferably about 50% or more. In some embodiments, the derivative according to the present invention may have water solubility improved by about 100% or more, about 200% or more, about 300% or more, about 400% or more, or about 500% or more, compared to lenalidomide or pomalidomide.

Meanwhile, in the present invention, it was confirmed that the lenalidomide or pomalidomide derivative can bind to cereblon (CRBN) with a binding affinity similar to that of lenalidomide or pomalidomide, thereby exhibiting an effect of degrading a CRBN substrate to a similar extent as lenalidomide or pomalidomide. In addition, it was confirmed that the lenalidomide or pomalidomide derivative can induce the death of multiple myeloma cells at a level similar to that of lenalidomide or pomalidomide. Moreover, in the present invention, in relation with teratogenicity, which is a side effect caused by decomposition of SALL4 during fetal development when a pregnant woman takes lenalidomide or pomalidomide, it was confirmed that the lenalidomide or pomalidomide derivative according to the present invention can reduce side effects compared to lenalidomide or pomalidomide by inducing less degradation of SALL4.

CRBN is an important and direct target of thalidomide and forms an E3 ubiquitin ligase complex with damaged DNA binding protein (DDB1), cullin-4A (CUL4A) and regulator of cullins 1 (ROC1). This complex allows ubiquitin to bind to specific proteins, making them targets for protein degradation. When CRBN is knocked down through RNA interference (RNAi), it has been confirmed that cell proliferation inhibition by lenalidomide or pomalidomide is blocked in various multiple myeloma cell lines, and it has been reported that lenalidomide or pomalidomide binds to CRBN via the glutarimide moiety to degrade its substrate, thereby exhibiting an anticancer effect.

In recent studies, it was confirmed that thalidomide and derivatives thereof (lenalidomide and pomalidomide; both are called immunomodulatory drugs (IMiDs)) are B cell survival factors and cause the degradation of transcription factors, such as Ikaros and Aiolos, halting the growth of multiple myeloma and changing the function of immune cells. Here, zinc finger transcription factors, such as Ikaros (IKZF1) and Aiolos (IKZF3), selectively bind to CRBN by IMiDs. In addition, it was revealed that, after direct binding of IMiDs to CRBN, an E3 ligase is activated, causing rapid ubiquitination and degradation of Ikaros and Aiolos (Science, 343, 305, 2014; Science, 343, 301, 2014). Ikaros and Aiolos are transcription factors for B cell and T cell differentiation, and toxic effects on multiple myeloma cells induce the death of myeloma cells by decreasing the expression of transcription factors such as IRF4 and Myc in B cells due to the loss of these two types of transcription factors.

Lenalidomide is the only IMiD approved for the treatment of myelodysplastic syndrome (MDS) in which chromosome 5q is deleted (5q-). However, its mechanism of action is not clear, and in 2015, in addition to Ikaros and Aiolos, casein kinase α (CK1α) was identified as a lenalidomide-dependent CRBN neosubstrate. 5q- MDS cells carry a chromosomal region including a deleted CSNK1A1 gene, resulting in haploinsufficient expression of CK1α. In these 5q- MDS cells, CK1α degradation by lenalidomide leads to cell death. In addition, CK1α degradation by lenalidomide is significantly stronger than that induced by thalidomide or pomalidomide, suggesting that it depends on a substrate ligand recognized by CRBN.

Meanwhile, in 2018, two independent groups reported that spalt like transcription factor 4 (SALL4), which is a C2H2 zinc finger transcription factor, is a thalidomide-dependent neosubstrate of CRBN. First, Fischer's research team identified substrates that decreased during treatment with thalidomide, lenalidomide, or pomalidomide by mass spectrometry using human embryonic stem cells (hESCs). SALL4 was identified as a causative gene for genetic disorders such as Duane-radial ray syndrome (Okihiro syndrome) and Holt-Oram syndrome, which partially overlap thalidomide embryopathy. In addition, Chamberlain's group also independently identified SALL4 based on structural similarity with known zinc finger-type neosubstrates, and both groups concluded that SALL4 is a substrate that causes the teratogenic effect of thalidomide-based compounds.

Therefore, in another aspect, the present invention relates to a pharmaceutical composition for preventing or treating cancer, which includes the derivative as an active ingredient.

In still another aspect, the present invention relates to a method of preventing or treating cancer, which includes administering the derivative to a subject in need thereof.

In yet another aspect, the present invention relates to the use of the derivative for preventing or treating cancer.

In yet another aspect, the present invention relates to the use of the derivative for preparing a drug for preventing or treating cancer.

In the present invention, the cancer may be blood cancer or solid cancer, but the present invention is not limited thereto.

In the present invention, the blood cancer may be acute leukemia, chronic leukemia, multiple myeloma, Hodgkin's lymphoma, or non-Hodgkin's lymphoma, but the present invention is not limited thereto.

In yet another aspect, the present invention relates to a pharmaceutical composition for preventing or treating a myelodysplastic syndrome, which includes the derivative as an active ingredient.

In yet another aspect, the present invention relates to a method of preventing or treating a myelodysplastic syndrome, which includes administering the derivative to a subject in need thereto.

In yet another aspect, the present invention relates to the use of the derivative for preventing or treating a myelodysplastic syndrome.

In yet another aspect, the present invention relates to the use of the derivative for preparing a drug for preventing or treating a myelodysplastic syndrome.

In yet another aspect, the present invention relates to a pharmaceutical composition for preventing or treating tumors, which includes the derivative as an active ingredient, wherein the tumors include benign tumors and malignant tumors.

In yet another aspect, the present invention relates to a method of preventing or treating tumors, which includes administering the derivative to a subject in need thereof.

In yet another aspect, the present invention relates to the use of the derivative for preventing or treating tumors.

In yet another aspect, the present invention relates to the use of the derivative for preparing a drug for preventing or treating tumors.

Meanwhile, in the present invention, it was confirmed that the lenalidomide or pomalidomide derivative can exhibit an anti-inflammatory effect which is similar to or better than lenalidomide or pomalidomide.

Therefore, in yet another aspect, the present invention relates to a pharmaceutical composition for preventing or treating an inflammatory disease, which includes the derivative as an active ingredient.

In yet another aspect, the present invention relates to a method of preventing or treating an inflammatory disease, which includes administering the derivative to a subject in need thereof.

In yet another aspect, the present invention relates to the use of the derivative for preventing or treating an inflammatory disease.

In yet another aspect, the present invention relates to the use of the derivative for preparing a drug for preventing or treating an inflammatory disease.

In the present invention, the inflammatory disease may be selected from the group consisting of psoriasis, rheumatoid arthritis, and Crohn's disease, but the present invention is not limited thereto.

In yet another aspect, the present invention relates to a pharmaceutical composition for preventing or treating a brain disease, which includes the derivative as an active ingredient.

In yet another aspect, the present invention relates to a method of preventing or treating a brain disease, which includes administering the derivative to a subject in need thereof.

In yet another aspect, the present invention relates to the use of the derivative for preventing or treating a brain disease.

In yet another aspect, the present invention relates to the use of the derivative for preparing a drug for preventing or treating a brain disease.

In the present invention, the brain disease may be selected from the group consisting of traumatic brain injury, Alzheimer's disease, Parkinson's disease, Huntington's disease, Lou Gehrig's disease, multiple system atrophy, Alzheimer's dementia, vascular dementia, frontotemporal dementia, Lewy dementia, ischemic stroke, hemorrhagic stroke, and multiple sclerosis, but the present invention is not limited thereto.

In addition, in the present invention, it was confirmed through animal experiments that the lenalidomide or pomalidomide derivative according to the present invention can exhibit an effect of effectively treating macular degeneration, which is one of the angiogenic diseases.

Accordingly, in yet another aspect, the present invention relates to a pharmaceutical composition for preventing or treating an angiogenic disease, which includes the derivative as an active ingredient.

In yet another aspect, the present invention relates to a method of preventing or treating an angiogenic disease, which includes administering the derivative to a subject in need thereof.

In yet another aspect, the present invention relates to the use of the derivative for preventing or treating an angiogenic disease.

In yet another aspect, the present invention relates to the use of the derivative for preparing a drug for preventing or treating an angiogenic disease.

In the present invention, the angiogenic disease may be selected from the group consisting of corneal transplant angiogenesis, angiogenic glaucoma, diabetic retinopathy, macular degeneration (e.g., exudative age-related macular degeneration or wet age-related macular degeneration), diabetic macular edema, a corneal disease caused by new blood vessels, spot degeneration, pterygium, retinal degeneration, retrolental fibroplasia, granular conjunctivitis, hemangioma, angiofibroma, vascular malformation, arteriosclerosis, vascular adhesions, and edematous sclerosis, but the present invention is not limited thereto.

As an exemplary embodiment, the angiogenic disease may be macular degeneration, and the derivative may be administered by intravitreal injection or injected into the eye in the form of eye drops.

The lenalidomide or pomalidomide derivative according to the present invention may be formed into a lenalidomide or pomalidomide form by cleaving a sugar or sugar derivative due to a pH change, thereby exhibiting a pharmaceutical effect. In one aspect, the lenalidomide or pomalidomide derivative may be prepared into a composition of about pH 7 to 8, and in a stage before being injected into the body, it may be injected into the body after changing the pH to pH 7 or less, .e.g., pH 6.5 or less, and preferably, pH 6 or less. In another aspect, the lenalidomide or pomalidomide derivative may be prepared into a composition of about pH 7 to 8 and then injected into the body, and then a sugar or sugar derivative may be cleaved as the pH changes in the body (e.g., as the pH decreases in an organ such as the stomach) to pH 7 or less, e.g., pH 6.5 or less, and preferably, pH 6 or less. However, the pharmacological mechanism of the derivative is not limited thereto.

As an exemplary embodiment, the present invention includes a pharmaceutical composition, which includes an effective amount of the lenalidomide or pomalidomide derivative according to the present invention as an active ingredient. In some embodiment, the pharmaceutical composition may be used to treat blood cancer (e.g., multiple myeloma, or non-Hodgkin's lymphoma), solid cancer, a myelodysplastic syndrome, an inflammatory disease (e.g., psoriasis, rheumatoid arthritis, or Crohn's disease), a brain disease (e.g., traumatic brain injury, Alzheimer's disease, Parkinson's disease, Huntington's disease, Lou Gehrig's disease, multiple system atrophy, Alzheimer's dementia, vascular dementia, frontotemporal dementia, Lewy dementia, ischemic stroke, hemorrhagic stroke, or multiple sclerosis), or an angiogenic disease (e.g., corneal transplant angiogenesis, angiogenic glaucoma, diabetic retinopathy, macular degeneration, diabetic macular edema, a corneal disease caused by new blood vessels, spot degeneration, pterygium, retinal degeneration, retrolental fibroplasia, granular conjunctivitis, a hemangioma, angiofibroma, vascular malformation, arteriosclerosis, vascular adhesion, or edematous sclerosis).

In the present invention, the term "pharmaceutical composition" or "pharmaceutical formulation" refers to a mixture containing a pharmaceutically acceptable excipient such as a diluent or carrier that make a novel compound of the present invention particularly suitable for *in vivo* or *ex vivo* diagnostic or therapeutic use. According to some embodiments, a pharmaceutical composition that includes the composition of the present invention may be provided by administering it to a subject as needed. In some embodiments, the composition of the present invention may be administered to humans.

In the present invention, the "combination administration" or "co-administration" means that a composition described herein is administered simultaneously, immediately before or after the administration of one or more additional therapies (e.g., an anticancer agent, chemotherapy, or treatment for an inflammatory disease or a brain disease). The compound of the present invention may be administered to a patient alone or in combination. Co-administration refers to the simultaneous or sequential administration of a compound individually or in combination (one or more compounds or agents). Therefore, a preparation may be combined with another active material if desired.

The "effective amount" or "therapeutically effective amount" used in the present invention refers to an amount of a compound or composition (e.g., a compound or composition of the present invention), which is sufficient to achieve a beneficial or desired result. An effective amount may be provided in one or more administrations, applications, or doses, and is not intended to be limited to a particular agent or administration route.

The description of the pharmaceutical composition provided herein principally relates to a pharmaceutical composition for administration to humans, but those of ordinary skill in the art will understand that such a composition is generally suitable for administration to all kinds of animals. That is, the pharmaceutical composition according to the present invention may also be administered to animals requiring veterinary care, for example, other mammals such as domestic animals (e.g., dogs, cats, etc.), farm animals (e.g., cattle, sheep, pigs, horses, etc.), and laboratory animals (e.g., rats, mice, guinea pigs, etc.). Experienced veterinary pharmacologists with a good understanding of the modifications of the pharmaceutical composition for administration to various animals can design and/or execute such modifications simply by routine experimentation if needed.

The pharmaceutical composition described herein may be prepared by any method known in the field of pharmacology or as discussed later in the text. Generally, such a preparation method includes mixing the active ingredient with an excipient and/or one or more other auxiliary ingredients, and if needed or desired, forming and/or packaging the resulting product into desired single- or multi-dose units.

The pharmaceutical composition of the present invention may be manufactured, packaged, and/or sold unpackaged in a single unit dose and/or a plurality of single unit doses. As used in the present invention, "unit dose" is an individual amount of pharmaceutical composition including a predetermined amount of active ingredient. The amount of active ingredient is generally equal to the dose of active ingredient administered to a subject and/or a convenient fraction of such a dose, for example, 1/2 or 1/3 of the dose.

The relative amounts of the active ingredient, pharmaceutically acceptable excipients, and/or any additional ingredient in the pharmaceutical composition of the present invention will vary depending on the identity, size, and/or disorder of a subject to be treated, and an administration route of the composition. As an example, the composition may include 0.001 to 100 %(w/w) of active ingredient.

As used herein, the pharmaceutically acceptable excipients include all solvents, dispersion media, diluents, or other liquid vehicles, dispersions, suspension aids, surfactants, isotonic agents, thickeners, or emulsifiers, preservatives, solid binders, and lubricants, which are suitable for a particular dosage form. Remington's literature [The Science and Practice of Pharmacy, 21st Edition, A. R. Gennaro, (Lippincott, Williams & Wilkins, Baltimore, MD, 2006] discloses various excipients used in the preparation of pharmaceutical compositions and known techniques for their preparation. Their use is considered to be within the scope of the present invention except for any conventional carrier or medium that cannot coexist with a material or derivative thereof by imparting an undesirable biological effect or interacting with other components of the pharmaceutical composition in a harmful manner. Pharmaceutically acceptable excipients are at least 95%, 96%, 97%, 98%, 99%, or 100% pure.

The excipients are approved for human and veterinary use. In some embodiments, the excipients are approved by the US FDA. In some embodiments, the excipients are pharmaceutical grade. In some embodiments, the excipients meet the standards of the United States Pharmacopoeia (USP), European Pharmacopoeia (EP), British Pharmacopoeia, and/or International Pharmacopoeia (EP).

The pharmaceutically acceptable excipients used in the preparation of the pharmaceutical composition may include inert diluents, dispersants, and/or granulating agents, surfactants and/or emulsifiers, disintegrants, binders, preservatives, buffers, lubricants, and/or oils, but the present invention is not limited thereto.

These excipients may be included in the formulations of the present invention. Excipients such as cocoa butter and suppository waxes, colorants, coating agents, sweeteners, flavoring agents, and perfuming agents may be included in the composition at the formulator's discretion.

Exemplary diluents include calcium carbonate, sodium carbonate, calcium phosphate, dicalcium phosphate, calcium sulfate, calcium hydrogen phosphate, sodium lactose phosphate, sucrose, cellulose, microcrystalline cellulose, kaolin, mannitol, sorbitol, inositol, sodium chloride, dry starch, corn starch, powdered sugar, and a combination thereof, but the present invention is not limited thereto.

Exemplary granulating agents and/or dispersing agents include potato starch, corn starch, tapioca starch, sodium starch glycolate, clay, alginic acid, guar gum, citrus pulp, agar, bentonite, cellulose and wood products, natural sponges, cation-exchange resins, calcium carbonate, silicate, sodium carbonate, cross-linked poly(vinyl-pyrrolidone) (crospovidone), sodium carboxymethyl starch (sodium starch gylcolate), carboxymethyl cellulose, cross-linked sodium carboxymethyl cellulose (croscarmellose), methylcellulose, pregelatinized starch (Starch 1500), microcrystalline starch, water-insoluble starch, calcium carboxymethyl cellulose, magnesium aluminum silicate (Veegum), sodium lauryl sulfate, quaternary ammonium compounds, and a combination thereof, but the present invention is not limited thereto.

Exemplary surfactants and/or emulsifiers include natural emulsifiers (e.g., acacia, agar, alginic acid, sodium alginate, tragacanth, chondrux, cholesterol, xanthan, pectin, gelatin, egg yolk, casein, wool fat, wax, and lecithin), colloidal clays (e.g., bentonite [aluminum silicate] and Veegum [magnesium aluminum silicate]), long-chain amino acid derivatives, high molecular weight alcohols (e.g., stearyl alcohol, cetyl alcohol, oleyl alcohol, triacetin monostearate, ethylene glycol distearate, glyceryl monostearate, and propylene glycol monostearate, and polyvinyl alcohol), carbomers (e.g., carboxy polymethylene, polyacrylic acid, acrylic acid polymers, and carboxyvinyl polymers), carrageenan, cellulose derivatives (e.g., carboxymethyl cellulose sodium, powdered cellulose, hydroxymethyl cellulose, hydroxypropyl cellulose, hydroxypropyl methylcellulose, and methyl cellulose), sorbitan fatty acid esters (e.g., polyoxyethylene sorbitan monolaurate [Tween 20], polyoxyethylene sorbitan [Tween 60], polyoxyethylene sorbitan monooleate [Tween 80], sorbitan monopalmitate [Span 40], sorbitan monostearate [Span 60], sorbitan tristearate [Span 65], glyceryl monooleate, sorbitan monooleate [Span 80]), polyoxyethylene esters (e.g., polyoxyethylene monostearate [Myrj 45], polyoxyethylene hydrogenated castor oil, polyethoxylated castor oil, polyoxymethylene stearate, and Solutol), sucrose fatty acid esters, polyethylene glycol fatty acid esters (e.g., Cremophor), polyoxyethylene ethers (e.g., polyoxyethylene lauryl ether [Brij 30]), poly(vinyl-pyrrolidone), diethylene glycol monolaurate, triethanolamine oleate, sodium oleate, potassium oleate, ethyl oleate, oleic acid, ethyl laurate, sodium lauryl sulfate, Pluronic F 68, Poloxamer 188, cetrimonium bromide, cetyl pyridinium chloride, benzalkonium chloride, docusate sodium, and/or a combination thereof, but the present invention is not limited thereto.

Exemplary binders include starches (e.g. corn starch and starch paste); gelatin; sugars (e.g. sucrose, glucose, dextrose, dextrin, molasses, lactose, lactitol, and mannitol); natural and synthetic gums (e.g. acacia, sodium alginate, Irish moss extract, panwar gum, ghatti gum, mucilage of isapgol husks, carboxymethylcellulose, methylcellulose, ethylcellulose, hydroxyethylcellulose, hydroxypropylcellulose, hydroxypropyl methylcellulose, microcrystalline cellulose, cellulose acetate, poly(vinyl-pyrrolidone), magnesium aluminum silicate (Veegum), and larch arabinogalactan); alginates; polyethylene oxides; polyethylene glycols; inorganic calcium salts; silicic acid; polymethacrylates; waxes; water; alcohols; and a combination thereof, but the present invention is not limited thereto.

Exemplary preservatives may include antioxidants, chelating agents, antimicrobial preservatives, antifungal preservatives, alcohol preservatives, acidic preservatives, and other preservatives. Exemplary antioxidants include alpha tocopherol, ascorbic acid, ascorbyl palmitate, butylated hydroxyanisole, butylated hydroxytoluene, monothioglycerol, potassium metabisulfite, propionic acid, propyl gallate, sodium ascorbate, sodium bisulfite, sodium metabisulfite, and sodium sulfite, but the present invention is not limited thereto. Exemplary chelating agents include ethylenediaminetetraacetic acid (EDTA), citric acid monohydrate, disodium edetate, dipotassium edetate, edetic acid, fumaric acid, malic acid, phosphoric acid, sodium edetate, tartaric acid, and trisodium edetate. Exemplary antimicrobial preservatives include benzalkonium chloride, benzethonium chloride, benzyl alcohol, bronopol, cetrimide, cetylpyridinium chloride, chlorhexidine, chlorobutanol, chlorocresol, chloroxylenol, cresol, ethyl alcohol, glycerin, hexetidine, imidurea, phenol, phenoxyethanol, phenylethyl alcohol, phenylmercuric nitrate, propylene glycol, and thimerosal, but the present invention is not limited thereto. Exemplary antifungal preservatives include butyl paraben, methyl paraben, ethyl paraben, propyl paraben, benzoic acid, hydroxybenzoic acid, potassium benzoate, potassium sorbate, sodium benzoate, sodium propionate, and sorbic acid, but the present invention is not limited thereto. Exemplary alcohol preservatives include ethanol, polyethylene glycol, phenol, phenol-based compounds, bisphenol, chlorobutanol, hydroxybenzoate, and phenylethyl alcohol, but the present invention is not limited thereto. Exemplary acidic preservatives include vitamin A, vitamin C, vitamin E, beta-carotene, citric acid, acetic acid, dehydroacetic acid, ascorbic acid, sorbic acid, and phytic acid, but the present invention is not limited thereto. Other preservatives include tocopherol, tocopherol acetate, deteroxime mesylate, cetrimide, butylated hydroxyanisole (BHA), butylated hydroxytoluende (BHT), ethylenediamine, sodium lauryl sulfate (SLS), sodium lauryl ether sulfate (SLES), sodium bisulfite, sodium metabisulfite, potassium sulfite, potassium metabisulfite, Glydant Plus, Phenonip, methyl paraben, Germall 115, Germaben II, Neolone, Kathon, and Euxyl, but the present invention is not limited thereto. In a specific embodiment, the preservative is an antioxidant. In another embodiment, the preservative is a chelating agent.

Exemplary buffers include citrate buffer solutions, acetate buffer solutions, phosphate buffer solutions, ammonium chloride, calcium carbonate, calcium chloride, calcium citrate, calcium glubionate, calcium gluceptate, calcium gluconate, D-gluconic acid, calcium glycerophosphate, calcium lactate, propanoic acid, calcium levulinate, pentanoic acid, dibasic calcium phosphate, phosphoric acid, tribasic calcium phosphate, calcium hydroxide phosphate, potassium acetate, potassium chloride, potassium gluconate, potassium mixtures, dibasic potassium phosphate, monobasic potassium phosphate, potassium phosphate mixtures, sodium acetate, sodium bicarbonate, sodium chloride, sodium citrate, sodium lactate, dibasic sodium phosphate, monobasic sodium phosphate, sodium phosphate mixtures, tromethamine, magnesium hydroxide, aluminum hydroxide, alginic acid, pyrogen-free water, isotonic saline, Ringer's solution, ethyl alcohol, and a combination thereof, but the present invention is not limited thereto.

Exemplary lubricants include magnesium stearate, calcium stearate, stearic acid, silica, talc, malt, glyceryl behenate, hydrogenated vegetable oils, polyethylene glycol, sodium benzoate, sodium acetate, sodium chloride, leucine, magnesium lauryl sulfate, sodium lauryl sulfate, and a combination thereof, but the present invention is not limited thereto.

Exemplary natural oils include almond, apricot kernel, avocado, babassu, bergamot, black current seed, borage, cade, camomile, canola, caraway, carnauba, castor, cinnamon, cocoa butter, coconut, cod liver, coffee, corn, cotton seed, emu, eucalyptus, evening primrose, fish, flaxseed, geraniol, gourd, grape seed, hazel nut, hyssop, isopropyl myristate, jojoba, kukui nut, lavandin, lavender, lemon, litsea cubeba, macadamia nut, mallow, mango seed, meadowfoam seed, mink, nutmeg, olive, orange, orange roughy, palm, palm kernel, peach kernel, peanut, poppy seed, pumpkin seed, rapeseed, rice bran, rosemary, safflower, sandalwood, sasquana, savory, sea buckthorn, sesame, shea butter, silicone, soybean, sunflower, tea tree, thistle, tsubaki, vetiver, walnut, and wheat germ oils, but the present invention is not limited thereto. Exemplary synthetic oils include butyl stearate, caprylic triglyceride, capric triglyceride, cyclomethicone, diethyl sebacate, dimethicone 360, isopropyl myristate, mineral oil, octyldodecanol, oleyl alcohol, silicone oil, and a combination thereof, but the present invention is not limited thereto.

Liquid dosage forms for oral and parenteral administrations include, but are not limited to, pharmaceutically acceptable emulsions, microemulsions, solutions, suspensions, syrups, and elixirs. In addition to the active ingredient, liquid formulations may include inert diluents commonly used in the art, for example, water or other solvents, solubilizers, and emulsifiers, such as ethyl alcohol, isopropyl alcohol, ethyl carbonate, ethyl acetate, benzyl alcohol, benzyl benzoate, propylene glycol, 1,3-butylene glycol, dimethylformamide, oil (particularly, cottonseed, peanut oil, corn oil, sprout oil, olive oil, castor oil, or sesame oil), glycerol, tetrahydrofurfuryl alcohol, polyethylene glycol of sorbitan, fatty acid esters, and a mixture thereof. Besides inert diluents, an oral composition may include an adjuvant such as a wetting agent, an emulsifier, a suspending agent, a sweetener, a flavoring agent, or a perfuming agent. In a specific embodiment for parenteral administration, the novel compound of the present invention is mixed with a solubilizer such as Cremophor, alcohol, oil, denatured oil, glycol, polysorbate, cyclodextrin, a polymer, or a combination thereof.

The lenalidomide or pomalidomide derivative according to the present invention may have significantly improved water solubility compared to its base, lenalidomide or pomalidomide, and may be used as an injection preparation in one embodiment.

Therefore, in yet another aspect, the present invention relates to an injection preparation, which includes the derivative.

In the present invention, the term "injection preparation" may be interchangeably used with a `formulation for injection,' an 'injection,' or an 'injectable preparation'

The injectable preparation, for example, a sterile injectable aqueous or oily suspension may be prepared according to the known art using a dispersing or wetting agent, and a suspending agent. A sterile injectable preparation may be a sterile injectable solution, suspension, or emulsion in a parenterally acceptable non-toxic diluent or solvent, for example, a solution in 1,3-butanediol. Those that can be selected among acceptable vehicles and solvents are water, Ringer's solution, U.S.P., and an isotonic sodium chloride solution. In addition, a sterile fixed oil is typically employed as a solvent or suspending medium. To this end, any bland fixed oil containing synthetic mono- or di-glycerides may be employed. In addition, a fatty acid, for example, oleic acid, is used in the preparation of an injection.

An injectable preparation may be sterilized, for example, by filtration through a bacteria-retaining filter, or by adding a sterilizing agent in the form of a sterile solid composition capable of being dissolved or dispersed in a sterile water or another sterile injectable medium before use.

In addition, the lenalidomide or pomalidomide derivative according to the present invention may have significantly improved water solubility compared to its base, lenalidomide or pomalidomide, and may be used as an eye drop preparation in an embodiment.

Therefore, in yet another aspect, the present invention relates to an eye drop preparation including the derivative.

In the present invention, the term "eye drop preparation" may be interchangeably used with an `eye drop formulation',' `eye drops,' an `ophthalmic drug preparation,' an `ophthalmic drug formulation,' or an 'ophthalmic drug.'

Meanwhile, to prolong the effect of a drug, it is often preferable to make the absorption of a drug from subcutaneous or intramuscular injection slower. This is achieved by using a liquid suspension of a crystalline or amorphous material with low water solubility. The absorption rate of the drug then depends on a dissolution rate, which may ultimately depend on a crystal size and a crystalline form. Alternatively, the delayed absorption of a drug for parenteral administration is achieved by dissolving or suspending the drug in an oil vehicle.

Solid dosage forms for oral administration include capsules, tablets, pills, powders, and granules. In such solid formulations, an active ingredient is mixed with one or more inert pharmaceutically acceptable excipients or carriers, such as sodium citrate or calcium diphosphate, and/or a) a filler or extender, such as starch, lactose, sucrose, glucose, mannitol, and silicic acid, b) a binder such as carboxymethylcellulose, alginate, gelatin, polyvinylpyrrolidinone, sucrose, and acacia, c) a humectant such as glycerol, d) a disintegrant such as agar, calcium carbonate, potato or tapioca starch, alginic acid, a specific silicate, and sodium carbonate, e) a dissolution retarding agent such as paraffin, f) an absorption accelerator such as a quaternary ammonium compound, g) a wetting agent such as cetyl alcohol and glycerol monostearate, h) an absorbent such as kaolin or bentonite clay, and i) a lubricant such as talc, calcium stearate, magnesium stearate, solid polyethylene glycol, sodium lauryl sulfate, and a mixture thereof.

The dosage forms such as capsules, tablets, and pills may include a buffer. Similar types of solid compositions may be employed as fillers in soft- and hard-filled gelatin capsules that use lactose, milk sugar, or high molecular weight polyethylene glycol as an excipient. Solid dosage forms such as tablets, dragees, capsules, pills, and granules may be prepared with coatings or shells, such as enteric coatings and other coatings well known in the field of pharmaceutical formulation. These may be compositions that optionally include an opacifying agent, and release only an active ingredient, or preferentially release an active ingredient in specific part of the intestinal tract, optionally, in a delayed manner. Examples of embedding compositions that can be used include polymeric materials and waxes. Similar types of solid compositions may be employed as fillers in soft- and hard-filled gelatin capsules that use lactose, milk sugar, or high molecular weight polyethylene glycol as an excipient.

The active ingredient may be in a micro-encapsulated form with one or more of the excipients described above. Solid dosage forms such as tablets, dragees, capsules, pills, and granules may be prepared with coatings or shells, such as enteric coatings, controlled release coatings, and other coatings well known in the field of pharmaceutical formulation. In these solid formulations, the active ingredient may also be mixed with one or more inert diluents such as sucrose, lactose, or starch. These formulations may contain additional materials other than inert diluents as in the ordinary embodiments, such as tablet lubricants, and other tablet auxiliaries such as magnesium stearate and microcrystalline cellulose. The dosage forms such as a capsule, a tablet, and a pill may include a buffer. These may optionally include an opacifying agent, or compositions that release only an active ingredient, or release an active ingredient preferentially, in a specific part of the intestinal tract, optionally, or in a delayed manner. An example of embedding composition that can be used includes a polymer material and a wax.

Dosage forms for topical and/or transdermal administration of the novel compound of the present invention or a pharmaceutical composition containing the same may include ointments, pastes, creams, lotions, gels, powders, solutions, sprays, inhalants, and/or patches. Generally, the active ingredient is mixed with a pharmaceutically acceptable carrier and/or any necessary preservative and/or a buffer that may be required under sterile conditions. In addition, the present invention considers the use of a transdermal patch, which often has an additional advantage of providing controlled delivery of the active ingredient to the body. Such formulations may be prepared, for example, by dissolving and/or dispersing the active ingredient in a suitable medium. Alternatively or additionally, the rate may be controlled by providing a rate controlling membrane and/or dispersing the active ingredient in a polymer matrix and/or gel.

Preparations for topical administration may include liquid and/or semi-liquid preparations, for example, liniments, lotions, oil-in-water and/or water-in-oil emulsions, such as creams, ointments or pastes, and/or solutions, and/or suspensions, but the present invention is not limited thereto. Although the concentration of the active ingredient may be as high as the solubility limit of the active ingredient in a solvent, a topically administrable preparation may also include, for example, about 1 to 10% (w/w) active ingredient. Preparations for topical administration may further include one or more additional ingredients described herein.

For example, preparations for topical administration may be prepared in dosage forms known in the art, which are administered to the eye.

The novel compound of the present invention described herein or a pharmaceutical composition containing the same is typically prepared in a dosage unit form for easy administration and uniform administration. However, it will be understood that the total daily dosage of the composition of the present invention will be determined by an attending physician within the scope of reasonable medical judgment. A specific therapeutically effective dose level for any particular subject will depend on various factors including a disease, a disorder, or a disorder to be treated, and the severity of a disorder; the activity of the specific active ingredient employed; the specific composition employed; the age, weight, overall health, gender, and diet of a subject; the administration time, administration route, and excretion rate of the specific active ingredient employed; the duration of treatment; a drug used in combination or simultaneously with the specific active ingredient employed; and other factors well known in the medical field.

The novel compound of the present invention, a salt thereof, or a pharmaceutical composition thereof may be administered through any route. In some embodiments, the novel compound, a salt thereof, or a pharmaceutical composition thereof is administered via various routes, such as orally, intravenously, intramuscularly, intraarterially, intramedullarily, intrathecally, subcutaneously, intracerebroventricularly, transdermally, intradermally, rectally, intravaginally, intraperitoneally, topically (by a powder, an ointment, a cream, and/or droplets), mucosally, nasally, orally or enterally, sublingually; endotracheal instillation, bronchial instillation, and/or inhalation; and/or by an oral spray, nasal spray, and/or an aerosol. Specifically considered routes are permeably intravenous injection, local administration via blood and/or lymphatic supply, and/or direct administration into an affected area. Generally, the most appropriate route of administration will depend on various factors, including the properties of an agent (e.g., stability in the environment of the gastrointestinal tract), and the discomfort of a subject (e.g., whether a subject can tolerate oral administration).

In a specific embodiment, the novel compound of the present invention, a salt thereof, or a pharmaceutical composition thereof may be administered daily at a dosage level sufficient to deliver about 0.001 to 100 mg/kg, 0.01 to 50 mg/kg, 0.1 to 40 mg/kg, 0.5 to 30 mg/kg, 0.01 to 10 mg/kg, 0.1 to 10 mg/kg, or 1 to 25 mg/kg of the body weight of a subject at least once a day to obtain a desired therapeutic effect. A desired dosage may be delivered three times a day, twice a day, daily, every two days, every three days, weekly, every two weeks, every three weeks, or every four weeks. In a specific embodiment, the desired dosage may also be delivered via multiple administrations (e.g., 2, 3, 4, 5, 6, 7, 8, 9, 10, 11, 12, 13, or 14 times or more administrations).

It will be understood that the dose range described herein provides guidance for the administration of a pharmaceutical composition provided to an adult. For example, the amount that is administered to a child or adolescent may be determined by a physician or one skilled in the art and may be less than or the same as that administered to an adult. The exact amount of a derivative according to the invention required to achieve an effective amount will vary from subject to subject depending, for example, on the subject's species, age and overall disorder, severity of side effects or disorders, identity of the characteristic compound, mode of administration, etc.

It will be understood that the novel compound and pharmaceutical composition of the present invention can be used in combination therapy. A particular combination of treatments (therapeutic agents or procedures) to be used for combination therapy will determine the desired therapeutic effect to be achieved and the suitability of desired therapeutic agent and/or procedure.

The pharmaceutical composition of the present invention may be administered alone or in combination with one or more therapeutically active agents. As for "combination," although the following delivery method is within the scope of the present invention, it is not intended to imply that an agent must be administered at the same exact time and/or formulated for delivery together. The composition may be administered simultaneously with, prior to, or subsequent to one or more other desired therapeutic agents or medical procedures. Generally, each agent will be administered at a dose and/or time schedule determined for the agent. In addition, the present invention encompasses delivering a pharmaceutical composition of the present invention in combination with an agent that improves the bioavailability of the composition in the body, reduces and/or modifies its metabolism, inhibits the secretion thereof, and/or modifies the distribution thereof. It will be further understood that the novel compound and therapeutically active agent of the present invention used in this combination may be administered together as a single composition, or separately as different compositions.

For the specific combination used in the combination therapy, a desired therapeutic effect to be achieved and/or procedures including the derivative of the present invention, and/or the suitability of a therapeutically active agent will be considered. It will be understood that used combinations may achieve a desired effect on the same disorder (e.g., the novel compound of the present invention may be administered in combination with another therapeutically active agent used to treat the same disorder), and/or exhibit different effects (e.g., control of any side effects).

The "therapeutically active agent" used herein refers to any material that is used as a medicine to treat, prevent, delay, reduce, or improve a disorder, and a material that is used in treatment, including preventive and curative treatments.

In some embodiments, the other therapeutically active agent for co-administration is for treating multiple myeloma. In some embodiments, the other therapeutically active agent may be a proteasome inhibitor and/or an immune modifying drug. In the present invention, the other therapeutically active agent may be selected from the group consisting of dexamethasone, bortezomib, carfilzomib, melphalan, doxorubicin, and cyclophosphamide, but the present invention is not limited thereto.

In some embodiments, the pharmaceutical composition of the present invention may be administered in combination with any therapeutically active agent or procedure (e.g., surgery or radiotherapy), which is used to treat, reduce, improve, or alleviate one or more symptoms or characteristics, delay the onset thereof, inhibit the progression thereof, reduce the severity thereof, and/or reduce the occurrence rate.

In various embodiments, the present invention provides a kit for treating multiple myeloma in a subject suffering from multiple myeloma. In some embodiments, the kit includes i) instructions for administering a lenalidomide or pomalidomide derivative composition or pharmaceutical composition according to the present invention to a subject suffering from multiple myeloma, and ii) the lenalidomide or pomalidomide derivative composition or pharmaceutical composition according to the present invention. In some embodiments, the kit may include one or more unit dosage forms, which contain the same content of lenalidomide or pomalidomide derivative composition or pharmaceutical composition as described herein, effective in treating multiple myeloma in a subject. In some embodiments, the subject is a human patient.

In some embodiments, the kit further includes one or more selected from the group consisting of a sterile syringe, a sterile needle, a sterile IV bag, an infusion pump, or any combination thereof.

The term "about" used herein may be interpreted to mean approximately, roughly, or to a certain extent. When the term "about" is used with a numerical range, it is interpreted as modifying a corresponding range by extending the boundary above and below a specified numerical value. Generally, the term "about" is used herein to modify a numerical value above and below the specified value by a variance of 10%.

The "individual," "patient," and "subject" are used interchangeably, and encompass any animals including mammals, e.g., mice, rats, other rodents, rabbits, dogs, cats, pigs, cattle, sheep, horses, or primates including humans.

### Examples

Hereinafter, the present invention will be described in more detail with reference to examples. The examples are merely provided to describe the present invention more fully, and it will be obvious to those of ordinary skill in the art that the scope of the present invention is not limited to the following examples.

### Example 1. Ribose-Lenalidomide

### 1-1. Synthesis

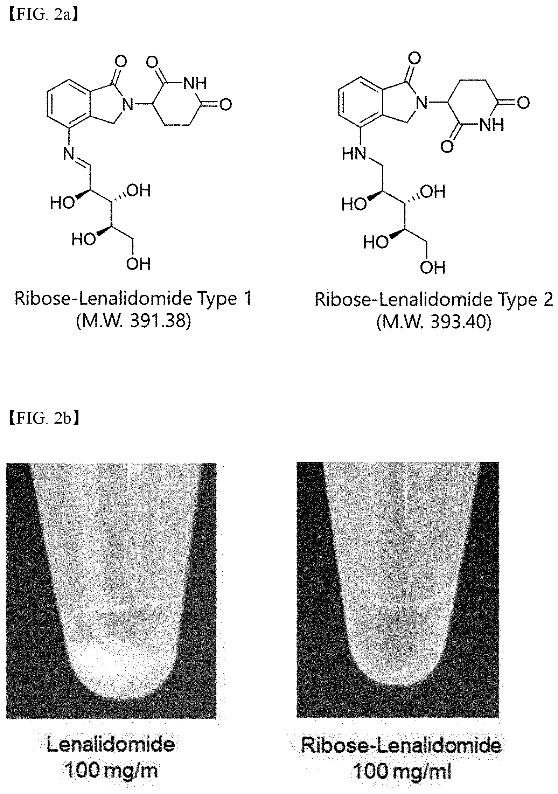

A solution prepared by dissolving 100 mg (0.386 mmol) of lenalidomide in 1.00 mL of diemthylformamide (DMF) and 1.00 mL of ethanol (EtOH) was mixed with a solution prepared by dissolving 290 mg (1.93 mmol) of ribose in 0.500 mL of water and 53.0 µL (0.926 mmol) of acetic acid (AcOH). The mixture was stirred at 60 °C overnight and concentrated under reduced pressure. Ribose-lenalidomide (Type 1, FIG. 2a, Formula V) was synthesized by purifying a residue through silicon oxide (SiO₂) column chromatography (dichloromethane (DCM) : methanol (MeOH) = 9 : 1 to 8 : 1), and then performing purification through C₁₈-SiO₂ column chromatography (methanol to 100% water ((MeOH): H₂O = 3 : 1)).

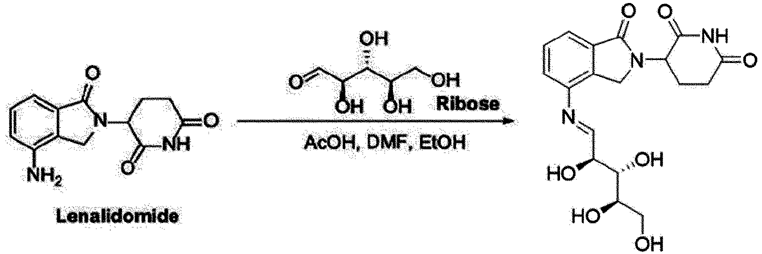

### 1-2. Water solubility

3 mg of lenalidomide (Combi-Block, Inc. San Diego, CA, USA) and ribose-lenalidomide were each weighed and placed in a 1.5 mL Eppendorf tube, 0.03 mL of water was added thereto, and then the mixture was vortexed for one minute and then centrifuged at 15,000 rpm for 1 minute, and an undissolved compound was precipitated. When the precipitated compound was visible, 0.03 mL of water was added, and the above process was repeated.

The solubility was determined by Table 1 as defined in United States Pharmacopeia 23 (USP23).

**[Table 1]**

| **Descriptive Term** | **Parts of Solvent Required for 1 part of Solute** | **g/L in water** | **M=400 mol/L in water** | **M=40000 mol/L in water** |
|---|---|---|---|---|
| Very soluble | ≤1 | ≥1000 | ≥2,5 | ≥0,025 |
| Freely soluble | 1 to 10 | 1000 to 100 | 2,5 to 0,25 | 0,025 to 0,0025 |
| Soluble | 10 to 30 | 100 to 33 | 0,25 to 0,08 | 0,0025 to 0,0008 |
| Sparingly soluble | 30 to 100 | 33 to 10 | 0,08 to 0,025 | 0,0008 to 0,00025 |
| Slightly soluble | 100 to 1000 | 10 to 1 | 0,025 to 0,0025 | 0,00025 to 0,0000025 |
| Very slightly soluble | 1000 to 10,000 | 1 to 0,1 | 0,0025 to 0,00025 | 0,000025 to 0,0000025 |
| Practically insoluble, or Insoluble | ≥10,000 | ≤0,1 | ≤0,00025 | ≤0,0000025 |

Consequently, as shown in FIG. 2b, it was confirmed that ribose-lenalidomide (type 1) has a solubility of 100 mg/mL or more in water (freely soluble based on USP 23), but lenalidomide is completely insoluble in water.

### 1-3. CRBN binding affinity

An immunomodulatory amide-type compound such as lenalidomide is known to act by binding to the cereblon (CRBN) protein. Therefore, the binding affinity of ribose-lenalidomide for the cereblon (CRBN) protein was compared with that of its base, lenalidomide (Combi-Block, Inc. San Diego, CA, USA), for the cereblon (CRBN) protein.

To this end, using a fluorescence-based Alpha assay kit (PROTAC Optimization kit for BET Bromodomain - Cereblon Binding (BPS Bioscience, #79770)), the competitive inhibition capacities of lenalidomide and ribose-lenalidomide for binding to CRBN and dBET1 were evaluated according to the manufacturer's instructions. In the experiment, AlphaLISA anti-FLAG acceptor beads, 5 mg/mL (PerkinElmer #AL112C), Alpha Glutathione donor beads, 5 mg/mL (PerkinElmer #6765300), Optiplate 384 (PerkinElmer #6007290), and L-Glutathione reduced (G4251, Sigma-Aldrich) were used.

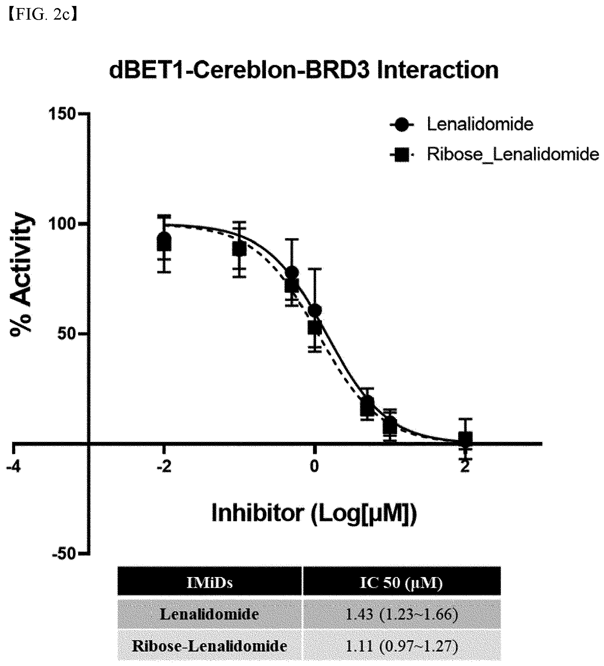

As a result, as shown in FIG. 2c, ribose-lenalidomide (type 1) was compared with lenalidomide to exhibit the improved binding affinity for the cereblon (CRBN) protein.

### 1-4. Tumor cell killing effect

Lenalidomide is an FDA-approved therapeutic agent for maintenance therapy after autologous stem cell transplantation for multiple myeloma patients, and it was intended to confirm whether ribose-lenalidomide also exhibits a multiple myeloma cell killing effect.

To this end, a human multiple myeloma cell line, MM.1S (CRL-2974), was purchased from the American Type Culture Collection (ATCC, Manassas, VA, USA), and incubated using a RPMI1640 medium (Corning, MD, USA) supplemented with a 10% bovine fetal serum (Corning, MD, USA), 100 U/mL penicillin (Corning, MD, USA), and 100 µg/mL streptomycin (Corning, MD, USA) at 37 °C in a 5% CO₂ incubator for 24 hours. The cells were seeded at 1 × 10⁴ cells/well in a 96-well plate, incubated for 24 hours, further incubated for 3 days after treatment with 0, 0.005, 0.014, 0.041, 0.123, 0.37, 1.111, 3.333, or 10 µM of ribose-lenalidomide dissolved in DMSO or D.W., and then analyzed using a CCK analysis kit (Dojindo, Japan) according to the manufacturer's instructions.

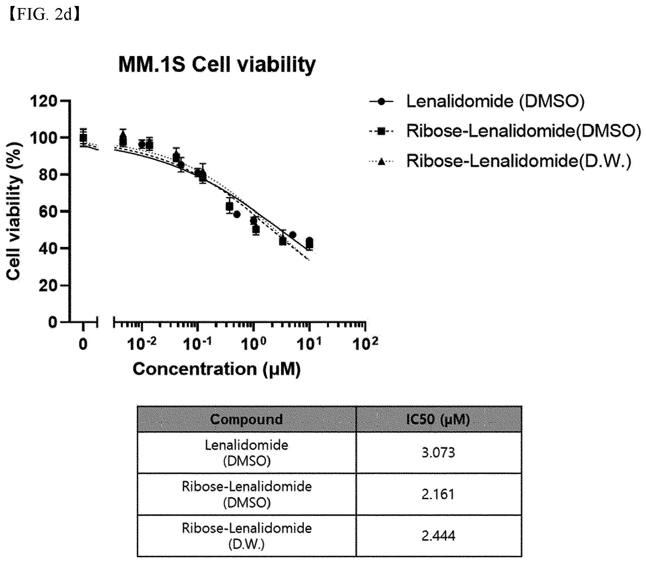

As a result, as shown in FIG. 2d, it was confirmed that ribose-lenalidomide (type 1) dissolved in DMSO or D.W. was effective in killing MM.1S cells, which is a human multiple myeloma cell line.

### 1-5. Aiolos, Ikaros, and CK1α degradation effects

It is known that a thalidomide-based compound binds to an E3 ligase, cereblon (CRBN), and decomposes transcription factors Aiolos and Ikaros as substrates, thereby regulating the function of immune cells. Therefore, to confirm whether ribose-lenalidomide still maintains its immunomodulatory function, the Aiolos and Ikaros degradation effect was compared with its base, lenalidomide.

In addition, CK1α known as another substrate of cereblon (CRBN) maintains oncogenic cascades such as PI3K/AKT, JAK/STAT and NF-κB in multiple myeloma and promotes stress-related signals that helps to combat various harmful effects. Therefore, it was intended to confirm whether ribose-lenalidomide can exhibit a multiple myeloma treatment effect by decomposing CK1α like lenalidomide.

To this end, a human multiple myeloma cell line, MM.1S (CRL-2974), was purchased from the American Type Culture Collection (ATCC, Manassas, VA, USA), and incubated using a DMEM medium (Corning, MD, USA) supplemented with 10% bovine fetal serum (Corning, MD, USA), 100 U/mL penicillin (Corning, MD, USA), and 100 µg/mL streptomycin (Corning, MD, USA) at 37 °C in a 5% CO₂ incubator.

After seeding the cells and incubating the cells for 24 hours, each compound was further incubated for 24 hours after treatment with 1 µM or 10 µM of each compound. The cells were lysed in a RIPA buffer containing a protease inhibitor cocktail (Thermo Fisher Scientific) and centrifuged at 14,000 rpm for 15 minutes at 4 °C, thereby obtaining a cell extract. The cell extract was loaded at the same amount, separated using SDS-PAGE, and transferred to a PVDF membrane. The protein-transferred membrane was blocked using skim milk, subjected to o/n incubation with a primary antibody at 4 °C, and incubated with an HRP-attached secondary antibody at 4 °C for 1 hour. The cells were washed with TBS-T three times between steps. Detection was performed by applying a chemiluminescence reagent (Thermo Fisher Scientific), and confirmation was performed using Chemidoc (iBright CL1000, Invitrogen, CA, USA). As the primary antibodies used, Aiolos (#15103), Ikaros (#9034), GAPDH(#2118S), and β-actin (#3700S) antibodies were purchased from Cell Signaling Technology (Danvers, MA, USA), and a CK1α (PA5-17536) antibody was purchased from Invitrogen (Waltham, Massachusetts USA).

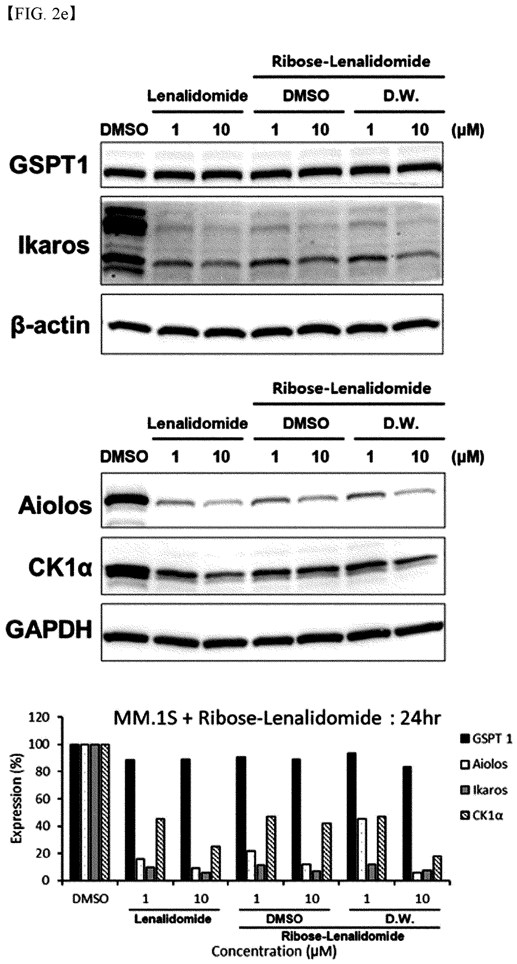

As a result, as shown in FIG. 2e, the Aiolos, Ikaros, and CK1α degradation effect of ribose-lenalidomide (type 1) was confirmed to be almost similar to lenalidomide, and the Aiolos, Ikaros, and CK1α degradation effect of ribose-lenalidomide did not show any difference depending on a solvent.

### 1-6. SALL4 degradation effect

It was reported that a cereblon (CRBN)-binding therapeutic agent may cause serious congenital abnormalities, such as forelimb shortening or brachydactyly, and this is closely related to SALL4 degradation. Therefore, the SALL4 degradation effect of ribose-lenalidomide was compared with lenalidomide.

To this end, a human embryonic carcinoma Tera-1 cell line (HTB-105) was purchased from the American Type Culture Collection (ATCC, Manassas, VA, USA), and incubated using a DMEM medium (Corning, MD, USA) supplemented with a 10% bovine fetal serum (Corning, MD, USA), 100 U/mL penicillin (Corning, MD, USA), and 100 µg/mL streptomycin (Corning, MD, USA) at 37 °C in a 5% CO₂ incubator.

The cells were seeded, incubated for 24 hours, and then further incubated after treatment with 1 or 10 µM of each of lenalidomide dissolved in DMSO and ribose-lenalidomide dissolved in DMSO or water. The cells were lysed by adding a RIPA buffer containing a protease inhibitor cocktail (Thermo Fisher Scientific), centrifuged at 14,000 rpm for 15 minutes at 4 °C, thereby obtaining a cell extract. The cell extract was loaded at the same amount, separated using SDS-PAGE, and transferred to a PVDF membrane. The protein-transferred membrane was blocked using skim milk, subjected to o/n incubation with a primary antibody at 4 °C, and incubated with an HRP-attached secondary antibody at 4 °C for 1 hour. The cells were washed with TBS-T three times between steps. Detection was performed by applying a chemiluminescence reagent (Thermo Fisher Scientific), and confirmation was performed using Chemidoc (iBright CL1000, Invitrogen, CA, USA). As the primary antibodies used, SALL4 (#5850) and GAPDH(#2118S) antibodies were purchased from Cell Signaling Technology (Danvers, MA, USA).

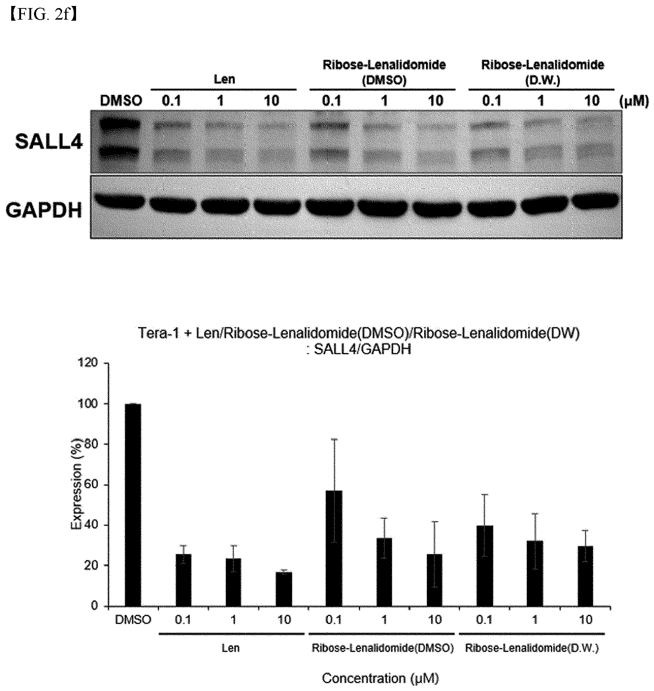

As a result, as shown in FIG. 2f, ribose-lenalidomide (type 1) has an inhibited SALL4 degradation effect compared to lenalidomide, showing that ribose-lenalidomide can improve teratogenic side effects compared to lenalidomide.

### 1-7. Anti-inflammatory effect

It was intended to compare the anti-inflammatory effects of ribose-lenalidomide and lenalidomide.

To this end, a human peripheral blood mononuclear cell line, PBMCs, was purchased from StemExpress (2.5 million, PBMNC025C, #1909040034), and stabilized using RPMI-1640 (Corning, MD, USA) supplemented with 10% bovine fetal serum (Corning, MD, USA), 100 U/mL penicillin (Corning, MD, USA), and 100 µg/mL streptomycin (Corning, MD, USA) at 37 °C in a 5% CO₂ incubator for 24 hours.

The cells were seeded at 1 × 10⁶ cells/well in a 96-well plate for 24 hours, treated with 0.1 or 1 µM of ribose-lenalidomide or lenalidomide, and after 1 hour, treated with PHA (25 µg/mL). After 6 hours, an RNA expression level was confirmed through real-time PCR, and 24 hours later, changes in inflammation-related cytokines were confirmed through ELISA.

RNA was isolated using an RNA prep kit (Monarch Total RNA Miniprep Kit, NEB) according to the manufacturer's instructions, cDNA was synthesized using a LunaScript RT SuperMix kit (NEB), and RT-PCR was performed using the following primer set.

**[Table 2]**

| Target | Primer sequences 5'->3' | Product size |
|---|---|---|
| TNF-α | F: CCCAGGGACCTCTCTCTAATC | 84 bp |
| | R: ATGGGCTACAGGCTTGTCACT | |
| IL-1β | F: ACAGATGAAGTGCTCCTTCCA | 73 bp |
| | R: GTCGGAGATTCGTAGCTGGAT | |
| IL-6 | F: GGCACTGGCAGAAAACAACC | 85 bp |
| | R: GCAAGTCTCCTCATTGAATCC | |
| IL-8 | F: AGAGTGATTGAGAGTGGACC | 118 bp |
| | R: ACTTCTCCACAACCCTCTG | |
| β-actin | F: GGATGCAGAAGGAGATCACTG | 90 bp |
| | R: CGATCCACACGGAGTACTTG | |

Products used in the experiment were RT-PCR cyber-green: Luna^{®} Universal qPCR Master Mix (M3003), Real-time PCR plate (48 well): MicroAmp^{®} Fast Optical 48-well Reaction Plate (4375816, Applied Biosystems), and Real-time PCR sealing film (48 well): MicroAmpTM 48-well Optical Adhesive Film (4375323, Applied Biosystems), and the experiment was conducted according to the manufacturer's instructions.

Meanwhile, after isolating the cell culture solution through centrifugation, the amount of TNF-α protein was analyzed using a human TNF alpha uncoated ELISA kit (88-7346-22, Invitrogen) according to the manufacturer's instructions.

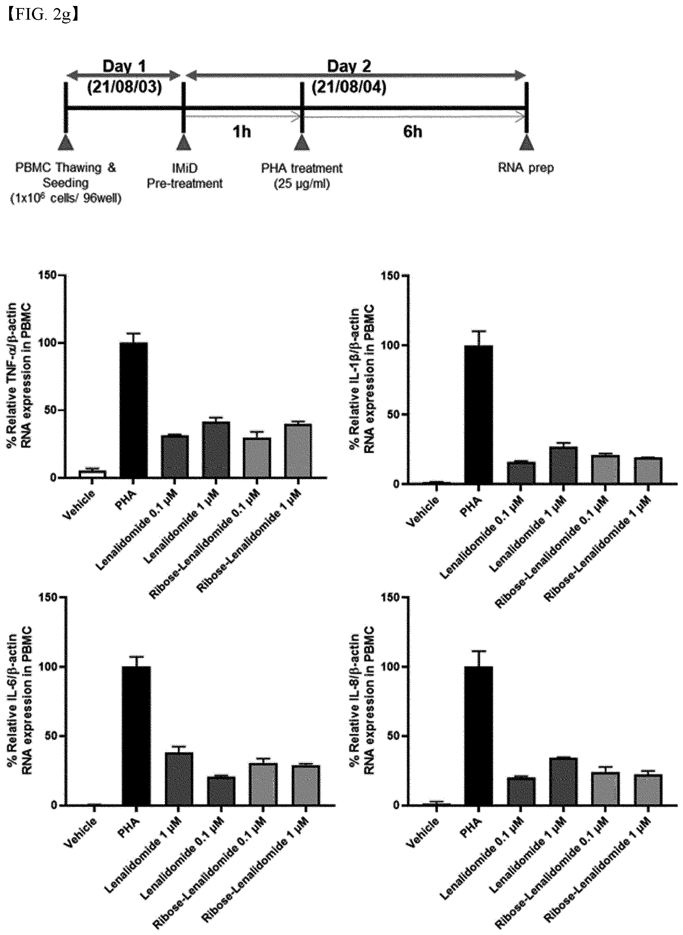

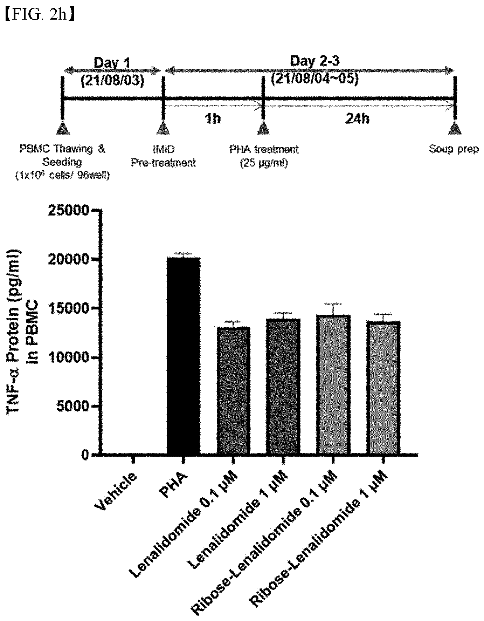

As a result, as shown in FIGS. 2G and 2H, it was confirmed that ribose-lenalidomide (type 1) exhibits an anti-inflammatory effect, which is similar to or slightly improved to that of lenalidomide at mRNA and protein levels.

### 1-8. Macular degeneration treatment effect

It was intended to confirm the healing efficacy of a lesion in a mouse CNV model, which is a wet senile macular degeneration (wet AMD) model.

To this end, five C57BL/6 (Koatech, Korea) male mice (aged 7 weeks) were used, and after 1 week of acclimation, the 8-week-old mice were anesthetized using ketamine hydrochloride (100 mg/kg, I.P.), and a drop of Mydriacyl (tropicamide) eye drops was placed on the right eye to induce mydriasis. After 3 minutes, the mouse was placed on a stand attached to a slit lamp, and a coverslip was put on the right eye to serve as a contact lens. After illuminating the optic nerve head using the slip lamp, four laser spots (200 mW, 50 µm spot size, 100-ms duration) around the optic nerve head were irradiated using a 532 nm laser device (Irdiex Oculight Tx, USA) attached to the slit lamp.

After forming a choroidal neovascularization lesion as described above, a solution prepared by dissolving ribose-lenalidomide in PBS was administered to the lesion. For intravitreal administration, 1.5 µL of the solution with a concentration of 0.2 µg/mL was administered once immediately after laser irradiation using a 25 µL Hamilton syringe with a 33G needle. Meanwhile, ribose-lenalidomide was dissolved in PBS to have a concentration of 2 mg/mL, prepared in the form of eye drops, administered four times a day, and then 7 days or 14 days after administration, efficacy was confirmed through fluorescent staining of blood vessels.

Fluorescent staining was performed using Heidelberg Spectralis HRA + OCT device (Heidelberg, Germany) equipment, the mouse was anesthetized using ketamine hydrochloride (100 mg/kg, I.P.) and xylazine (10 mg/kg, I.P.), a drop of Mydriacyl (tropicamide) eye drops was placed on the right eye to induce mydriasis, 0.05 mL of 10% fluorescite (Alcon, USA) was administered via I.P. administration to stain blood vessels. Fluorescent images were taken 10 minutes after fluorescite injection.

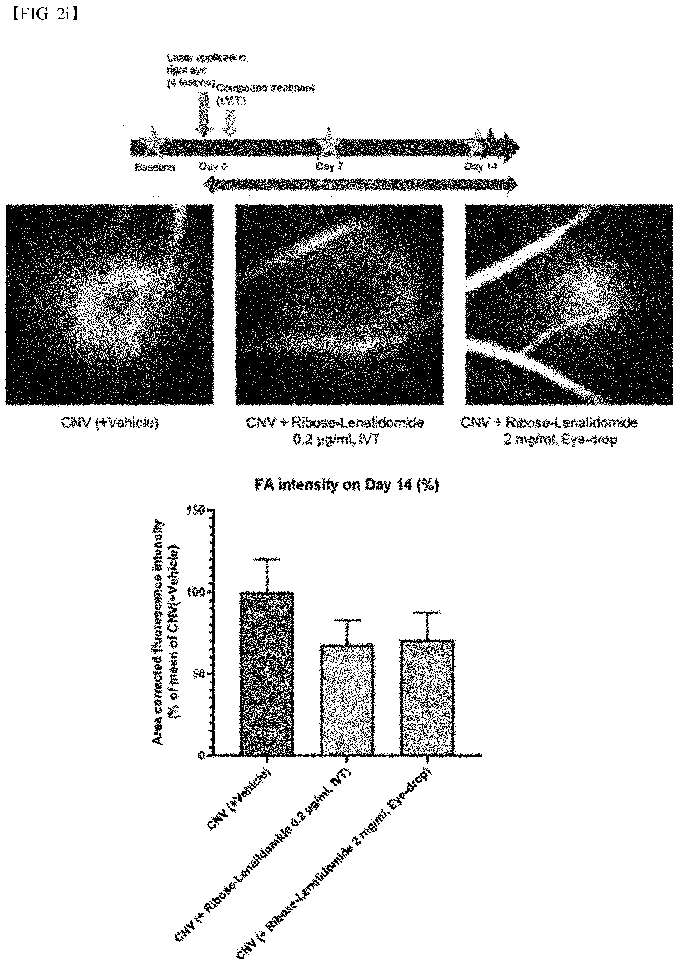

As a result, as shown in FIG. 2i, a distinct decrease in the lesion was confirmed in both the group in which ribose-lenalidomide (type 1) was intravitreally administered and the eye drop-administered group, compared to a control-administered group.

### Example 2. Glucose-lenalidomide

### 2-1. Synthesis

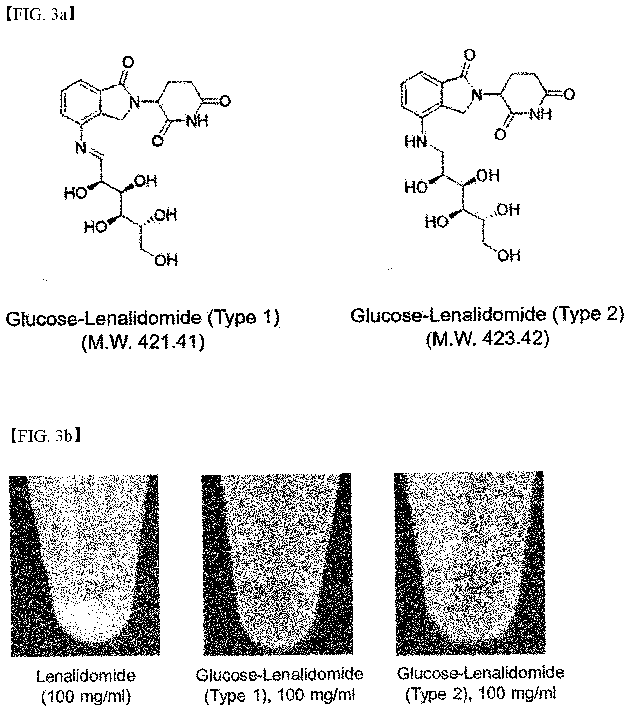

After dissolving 100 mg (0.386 mmol) of lenalidomide in 1.00 mL of dimethylformamide (DMF) and 1.00 mL of ethanol (EtOH), 208 mg (1.16 mmol) of glucose was dissolved in 0.50 mL of water (H₂O) and 53.0 µL (0.926 mmol) of acetic acid (AcOH) and then added. The mixture was stirred at 60 °C overnight and concentrated under reduced pressure. Glucose-lenalidomide (type 1, Formula III, FIG. 3a) was synthesized by purifying the residue through C₁₈-SiO₂ column chromatography (MeOH : 100% water = 3:1).

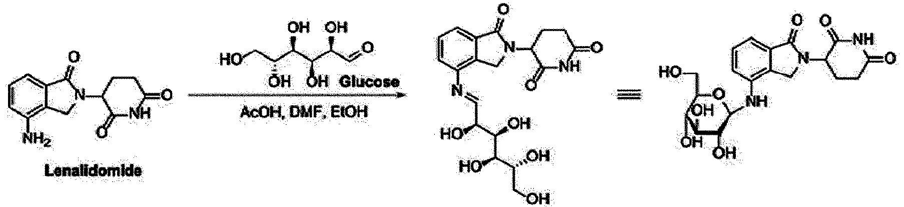

Meanwhile, 53.0 µL (0.926 mmol) of acetic acid (AcOH) and 58.2 mg (0.926 mmol) of sodium cyanoborohydride (NaBH₃CN) were added to a solution in which 200 mg (0.772 mmol) of lenalidomide and 167 mg (0.926 mmol) of glucose were dissolved in 2.00 mL of dimethylformamide (DMF) and 0.500 mL of water (H₂O). The mixture was stirred at 65 °C overnight. 1N HCl was added to bring a pH of the mixture to pH1 to 2. The mixture was diluted with 5.00 mL of water and washed with 5.00 mL of dimethylformamide (DMF) five times. The aqueous layer was concentrated under reduced pressure. Another type of glucose-lenalidomide (type 2, Formula IV, FIG. 3a) was synthesized by purifying a residue through C₁₈-SiO₂ column chromatography (MeOH : 100% water = 1 : 5).

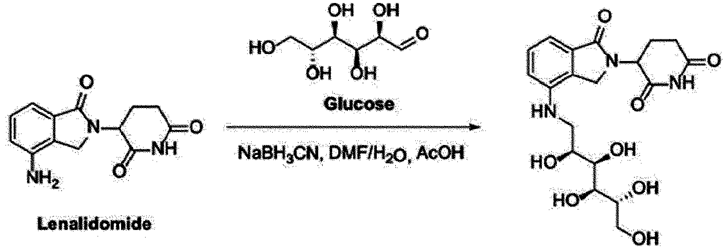

### 2-2. Water solubility

The water solubility of glucose-lenalidomide was confirmed by the same method as in Example 1-2.

As a result, as shown in FIG. 3b, it was confirmed that lenalidomide was barely dissolved in D.W., but the two types of glucose-lenalidomide show a solubility in D.W. of 100 mg/mL or more (freely soluble based on USP 23).

### 2-3. CRBN binding affinity

The CRBN binding affinity of glucose-lenalidomide was confirmed in the same manner as in Example 1-3.

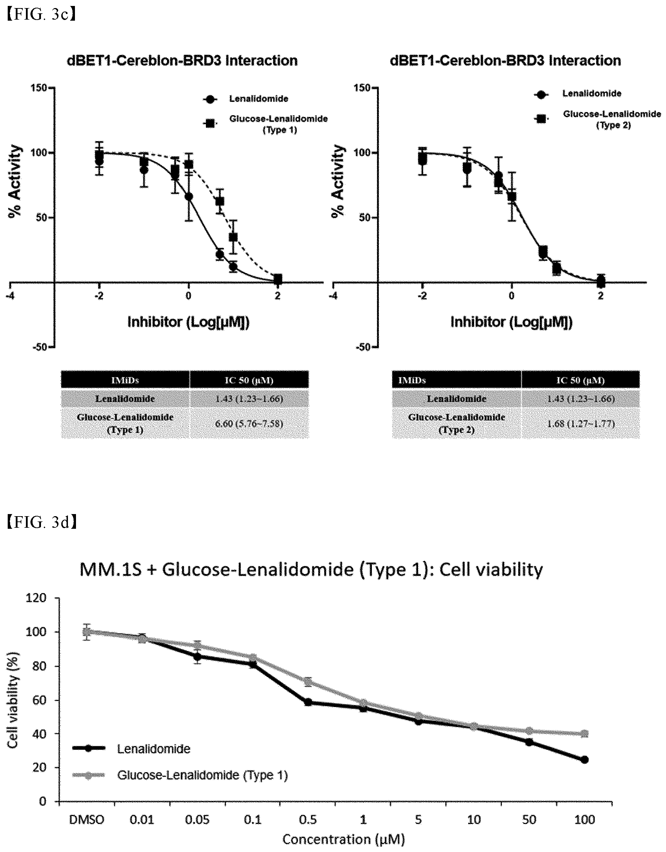

As a result, as shown in FIG. 3c, it was confirmed that the CRBN binding affinity of glucose-lenalidomide was slightly lower than or similar to that of lenalidomide.

### 2-4. Tumor cell killing effect

The tumor cell killing effect of glucose-lenalidomide was confirmed in the same manner as in Example 1-4.

As a result, as shown in FIG. 3d, it was confirmed that glucose-lenalidomide (type 1) has a similar effect of killing MM.1S cells, which is a human multiple myeloma cell line, to lenalidomide.

### 2-5. SALL4 degradation effect

The SALL4 degradation effect of glucose-lenalidomide was confirmed in the same manner as in Example 1-6.

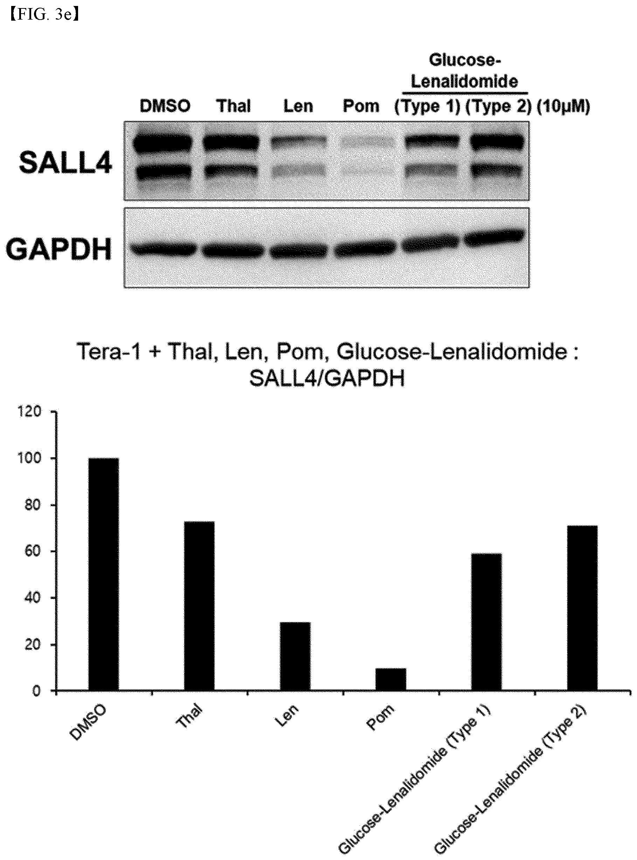

As a result, as shown in FIG. 3e, it was confirmed glucose-lenalidomide has a reduced possibility of teratogenic side effect due to a significant decrease in SALL4 degradation effect, compared to lenalidomide.

### 2-6. Macular degeneration treatment effect

The macular degeneration treatment effect of glucose-lenalidomide was confirmed by intravitreally administering the glucose-lenalidomide once in the same manner as in Example 1-8 using a mouse CNV model, which is a wet aged macular degeneration (wet AMD) model.

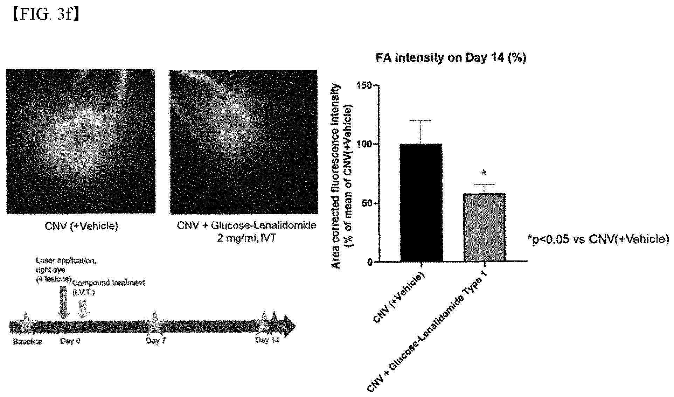

As a result, as shown in FIG. 3f, a macular degeneration treatment effect was confirmed through glucose-lenalidomide (type 1) administration.

### Example 3. Ribose-pomalidomide

### 3-1. Synthesis

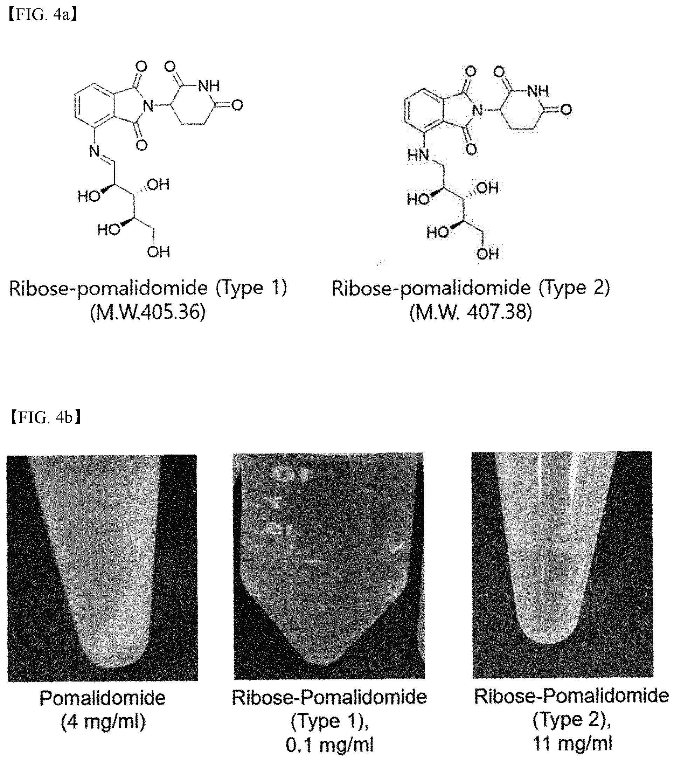

300 mg (1.16 mmol) of pomalidomide, 1.74 g (11.6 mmol) of ribose, and 9.00 mL of dioxane were dissolved in 0.75 mL of water, and then 53.0 µL (0.926 mmol) of acetic acid (AcOH) was added. The mixture was stirred at 100 °C overnight. A residue was diluted with 5.0 mL of water and washed with 10.0 mL of dimethylformamide (DMF) four times. An aqueous layer was concentrated under reduced pressure. A residue was purified through SiO₂ column chromatography (DCM : MeOH = 9 : 1), thereby obtaining 247 mg of ribose-pomalidomide (type 1, Formula IX, FIG. 4a).

247 mg of ribose-pomalidomide (type 1) was dissolved in 4.0 mL of water, 53.0 µL (0.926 mmol) of acetic acid (AcOH) and 76.6 mg (1.22 mmol) of sodium cyanoborohydride (NaBH₃CN) were added. The mixture was stirred at room temperature for 4.5 hours. 1N HCl was added to bring the pH of the mixture to pH1 to 2. The mixture was washed with 5.00 mL of dimethylformamide (DMF) three times. An aqueous layer was concentrated under reduced pressure. A residue was purified through SiO₂ column chromatography (DCM : MeOH = 8 : 2), and then purified through C₁₈-SiO₂ column chromatography (MeOH : 100% water: H₂O = 1 : 3 to 1 : 1). Fractions containing ribose-pomalidomide were collected, concentrated, and treated with n-hexane, thereby synthesizing 74.2 mg of ribose-pomalidomide (type 2, Formula X, FIG. 4a).

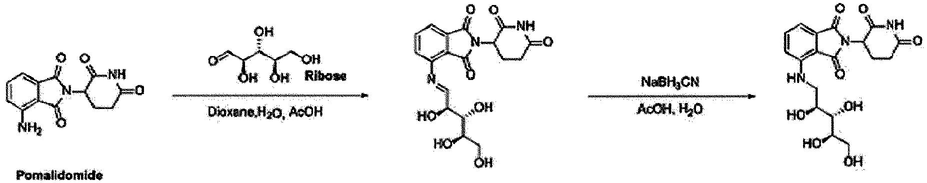

### 3-2. Water solubility

The water solubility of two types of ribose-pomalidomide was confirmed in the same manner as in Example 1-2.

As a result, as shown in FIG. 4b, it was confirmed that ribose-pomalidomide (type 1) continued to precipitate (practically insoluble or insoluble level based on USP 23) even at a concentration of 0.1 mg/mL or less, and it was confirmed that ribose-pomalidomide (type 2) was dissolved without visible precipitation (sparingly soluble level based on USP 23) at a concentration of 11 mg/mL.

### 3-3. CRBN binding affinity

The CRBN binding affinity of ribose-pomalidomide was confirmed in the same manner as in Example 1-3.

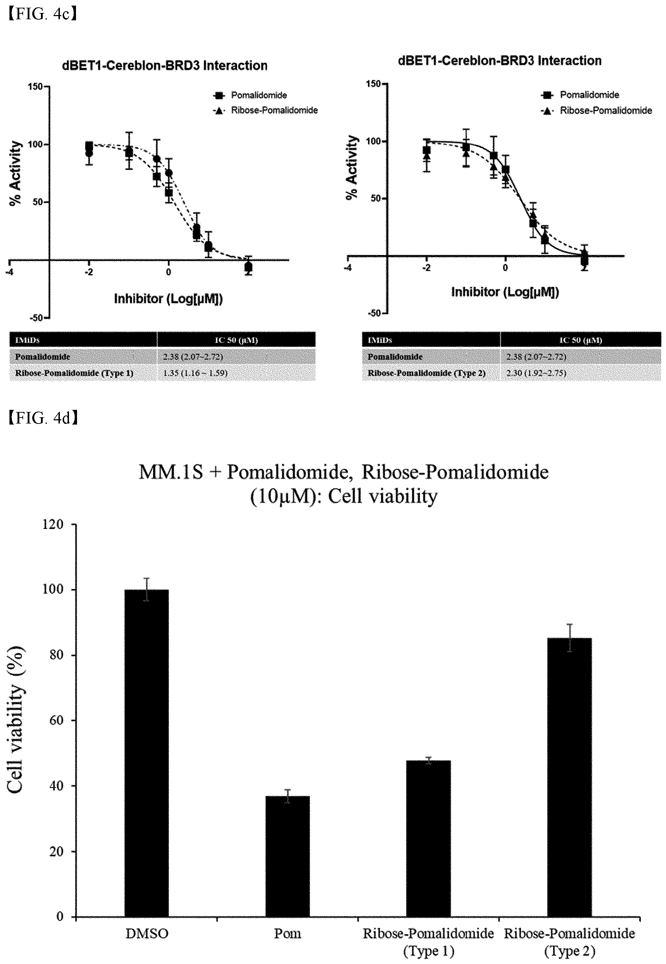

As a result, as shown in FIG. 4c, two types of ribose-pomalidomide showed similar or slightly improved CRBN binding affinity compared to pomalidomide.

### 3-4. Tumor cell killing effect

The tumor cell killing effect of ribose-pomalidomide was confirmed in the same manner as in Example 1-4.

As a result, as shown in FIG. 4d, it was confirmed that the viability of MM.1S cells, which is a human multiple myeloma cell line, was reduced by ribose-pomalidomide (type 1) but not significantly reduced by ribose-pomalidomide (type 2).

### 3-5. SALL4 degradation effect

The SALL4 degradation effect of ribose-pomalidomide was confirmed in the same manner as in Example 1-5.

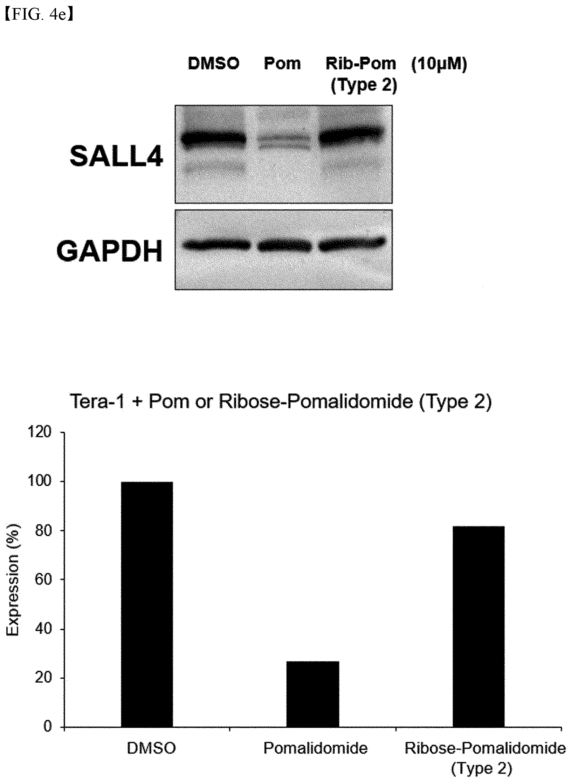

As a result, as shown in FIG. 4e, it was confirmed that ribose-pomalidomide (type 2) has significantly smaller SALL4 degradation effect than pomalidomide, reducing the possibility of teratogenic side effects.

### Example 4. Glucose-pomalidomide

### 4-1. Synthesis

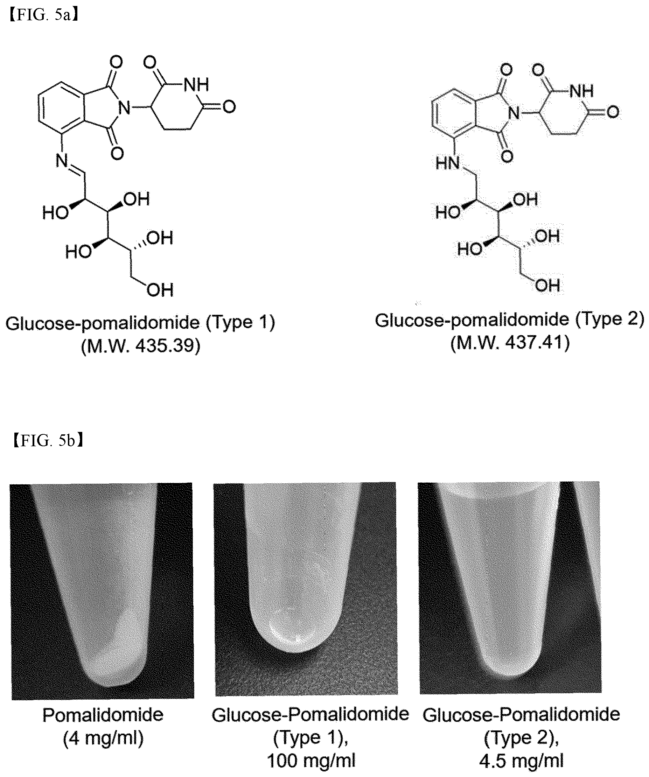

300 mg (1.16 mmol) of pomalidomide, 2.09 g (11.6 mmol) of glucose, and 9.00 mL of dioxane were dissolved in 0.75 mL of water, and then 53.0 µL (0.926 mmol) of acetic acid (AcOH) was added. The mixture was stirred at 100 °C overnight and concentrated under reduced pressure. A residue was diluted with 10.0 mL of water and washed with 10.0 mL of dimethylformamide (DMF) four times. An aqueous layer was concentrated under reduced pressure. A residue was purified through C₁₈-SiO₂ column chromatography (MeOH : 100% water: H2O = 3 : 1), thereby obtaining 96.8 mg of glucose-pomalidomide (type 1, Formula VII, FIG. 5a).

96.8 mg of glucose-pomalidomide (type 1) was dissolved in 1.5 mL of water, three drops of acetic acid (AcOH), and 27.9 mg (0.445 mmol) of sodium cyanoborohydride (NaBH₃CN) were added. The mixture was stirred at room temperature for 4.5 hours. 1N HCl was added to bring a pH of the mixture to pH1 to 2. The mixture was washed with 3.00 mL of dimethylformamide (DMF) three times. An aqueous layer was concentrated under reduced pressure. A residue was purified through SiO₂ column chromatography (DCM : MeOH = 8 : 2). Fractions containing ribose-pomalidomide (type 2) were collected, concentrated, and treated with n-hexane, thereby synthesizing 68.6 mg of glucose-pomalidomide (type 2, Formula VIII, FIG. 5a).

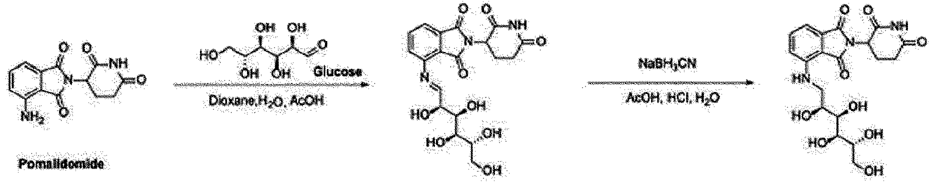

### 4-2. Water solubility

The water solubility of glucose-pomalidomide was confirmed in the same manner as in Example 1-2.

As a result, as shown in FIG. 5b, it was confirmed that the water solubility of the two types of glucose-pomalidomide was significantly improved compared to pomalidomide.

### 4-3. CRBN binding affinity

The CRBN binding affinity of glucose-pomalidomide was confirmed in the same manner as in Example 1-3.

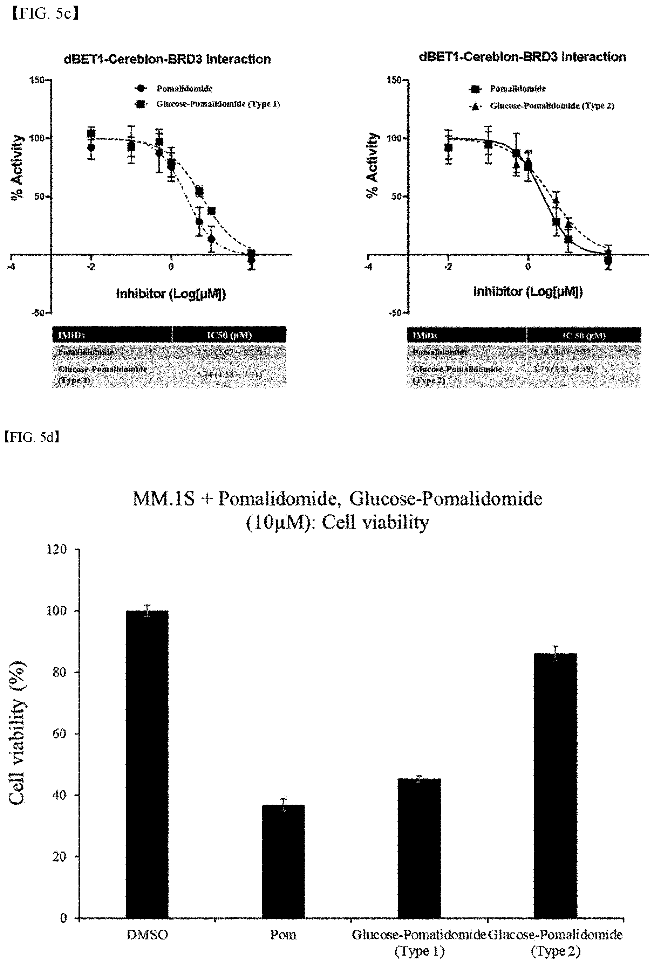

As a result, as shown in FIG. 5c, the CRBN binding affinity of glucose-pomalidomide was confirmed.

### 4-4. Tumor cell killing effect

The tumor cell killing effect of glucose-pomalidomide was confirmed in the same manner as in Example 1-4.

As a result, as shown in FIG. 5d, it was confirmed that the viability of MM.1S cells, which is a human multiple myeloma cell line, was reduced by glucose-pomalidomide (type 1), but not significantly reduced by glucose-pomalidomide (type 2).

As above, as specific parts of the present invention have been described in detail, it is clear to those skilled in the art that these specific techniques are merely preferred embodiments, and the scope of the present invention is not limited thereto. Thus, the actual scope of the present invention will be defined by the accompanying claims and their equivalents.

## Claims

1. A lenalidomide or pomalidomide derivative in which a sugar or sugar derivative is bound to an amino group (NH₂) on the phthalimide moiety of lenalidomide or pomalidomide.

2. The lenalidomide or pomalidomide derivative of claim 1, wherein the sugar or sugar derivative is a triose, a tetrose, a pentose, a hexose, or a derivative thereof.

3. The lenalidomide or pomalidomide derivative of claim 1, wherein the sugar or sugar derivative is glucose, ribose, glucuronic acid, or glycerin aldehyde.

4. The lenalidomide or pomalidomide derivative of claim 3, wherein the derivative is selected from the group consisting of Formulas III to XIV: 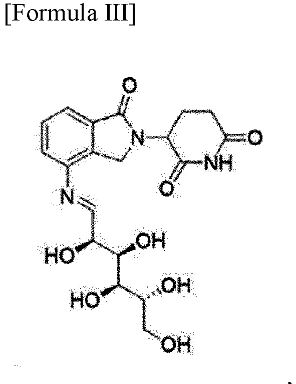 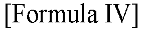 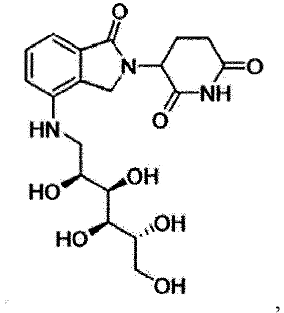 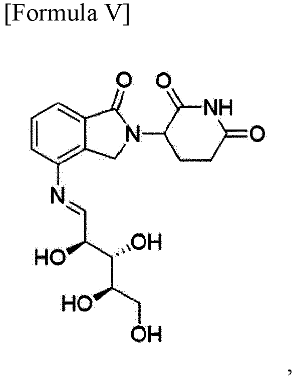 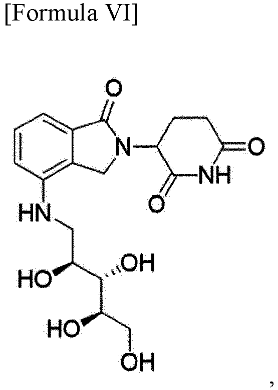 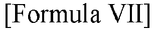 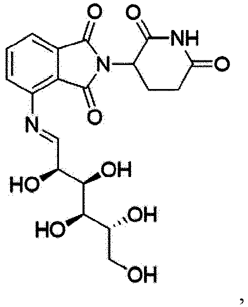 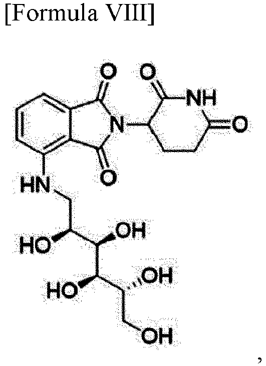 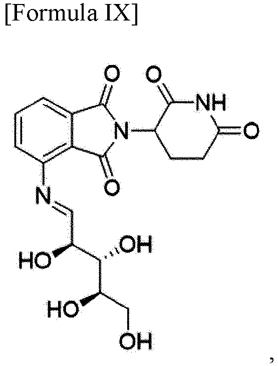 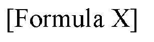 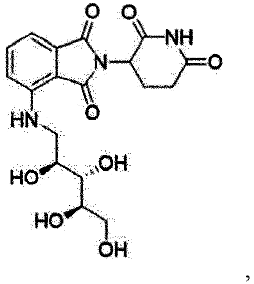 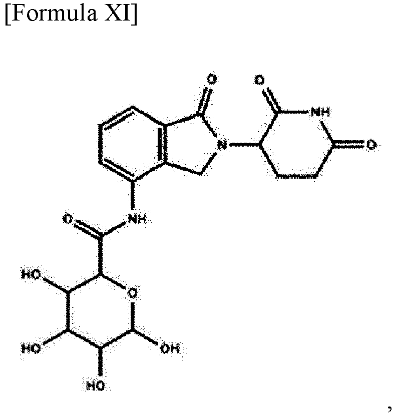 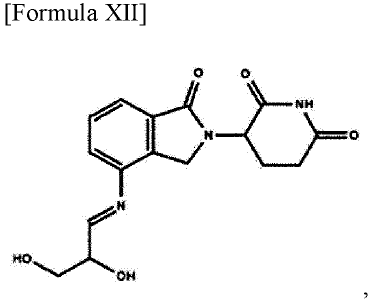 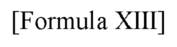 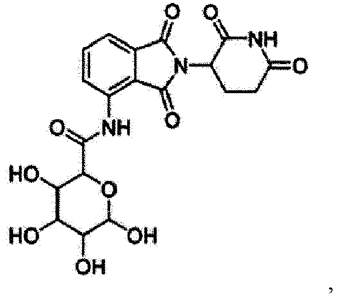 and

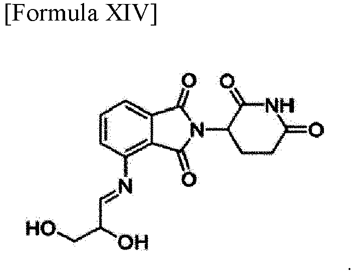

5. The lenalidomide or pomalidomide derivative of claim 1, wherein the derivative has 50% or more improved water solubility compared to lenalidomide or pomalidomide.

6. A pharmaceutical composition for preventing or treating cancer, comprising the derivative of any one of claims 1 to 5 as an active ingredient.

7. The composition of claim 6, wherein the cancer is blood cancer or solid cancer.

8. The composition of claim 7, wherein the blood cancer is selected from the group consisting of acute leukemia, chronic leukemia, multiple myeloma, Hodgkin's lymphoma, and non-Hodgkin's lymphoma.

9. A pharmaceutical composition for preventing of treating a myelodysplastic syndrome, comprising the derivative of any one of claims 1 to 5 as an active ingredient.

10. A pharmaceutical composition for preventing or treating an inflammatory disease, comprising the derivative of any one of claims 1 to 5 as an active ingredient.

11. The composition of claim 10, wherein the inflammatory disease is selected from the group consisting of psoriasis, rheumatoid arthritis, and Crohn's disease.

12. A pharmaceutical composition for preventing or treating a brain disease, comprising the derivative of any one of claims 1 to 5 as an active ingredient.

13. The pharmaceutical composition of claim 12, wherein the brain disease is selected from the group consisting of traumatic brain injury, Alzheimer's disease, Parkinson's disease, Huntington's disease, Lou Gehrig's disease, multiple system atrophy, Alzheimer's dementia, vascular dementia, frontotemporal dementia, Lewy dementia, ischemic stroke, hemorrhagic stroke, and multiple sclerosis.

14. A pharmaceutical composition for preventing or treating an angiogenic disease, comprising the derivative of any one of claims 1 to 5 as an active ingredient.

15. The composition of claim 14, wherein the angiogenic disease is selected from the group consisting of corneal transplant angiogenesis, angiogenic glaucoma, diabetic retinopathy, macular degeneration, diabetic macular edema, a corneal disease caused by new blood vessels, spot degeneration, pterygium, retinal degeneration, retrolental fibroplasia, granular conjunctivitis, a hemangioma, angiofibroma, vascular malformation, arteriosclerosis, vascular adhesion, and edematous sclerosis.

16. An injection preparation comprising the derivative of any one of claims 1 to 5 as an active ingredient.

17. An eye drop preparation comprising the derivative of any one of claims 1 to 5 as an active ingredient.
